# EUROPÄISCHE PATENTSCHRIFT

(11) **EP 1 694 306 B1**
(45) Veröffentlichungstag und Bekanntmachung des Hinweises auf die Patenterteilung: **12.09.2018**
(21) Anmeldenummer: 04803615.6
(22) Anmeldetag: 08.12.2004
(51) Int. Cl.: A61K 9/16

(54) **PULVER ENTHALTEND NIEDERMOLEKULARES DEXTRAN UND VERFAHREN ZU DEREN HERSTELLUNG**
POWDER CONTAINING LOW-MOLECULAR DEXTRAN AND METHOD FOR PRODUCING THE SAME
POUDRE CONTENANT UN DEXTRANE DE FAIBLE POIDS MOLÉCULAIRE ET PROCÉDÉ DE FABRICATION ASSOCIÉ

(30) Priorität: 13.12.2003 DE 10358387
(43) Veröffentlichungstag der Anmeldung: 30.08.2006
(73) Patentinhaber: Boehringer Ingelheim Pharma GmbH & Co. KG, 55216 Ingelheim am Rhein (DE)
(72) Erfinder: BECHTOLD-PETERS, Karoline, 88400 Biberach (DE); FRIESS, Wolfgang, 82393 Iffeldorf (DE); FUHRHERR, Richard, 90453 Nürnberg (DE); BASSARAB, Stefan, 88400 Biberach (DE); GARIDEL, Patrick, 22846 Norderstedt (DE); SCHULTZ-FADEMRECHT, Torsten, 88437 Maselheim, OT Äpfingen (DE)
(74) Vertreter: Simon, Elke Anna Maria
(86) Internationale Anmeldenummer: PCT/EP2004/013938
(87) Internationale Veröffentlichungsnummer: WO 2005/055976

(56) Entgegenhaltungen:
- EP-A- 1 184 032
- WO-A-03/094887
- LIAO YONG-HONG; BROWN MARC; NAZIR TAHIR; QUADER ABDUL AND MARTIN GARY: "Effects of sucrose and trehalose on the preservation of the native structure of spray-dried lysozyme" PHARMACEUTICAL RESEARCH, Bd. 19, Nr. 12, 2002, Seiten 1847-1853, XP008056896

## Beschreibung

### Gebiet der Erfindung

Die Erfindung betrifft Sprühgetrocknetes Pulver enthaltend einen pharmazeutischen Wirkstoff, ein Tri-Isoleucin und ein niedermolekulares Dextran mit einem Molekulargewicht zwischen 500 und 10.000 Da Ferner betrifft die vorliegende Erfindung Verfahren zur Herstellung sprühgetrockneten Pulvers sowie Verwendungen des sprühgetrockneten Pulvers zur Herstellung von Arzneimitteln.

### Hintergrund

In wässrigen Lösungen formulierte Wirkstoffe/Wirkstoffzubereitungen unterliegen zum Teil Instabilitäten, welche zu verminderter Bioaktivität und erhöhten Unverträglichkeiten führen können. Eine Möglichkeit der Stabilisierung bietet beispielsweise die Sprühtrocknung, bei der der pharmazeutische Wirkstoff durch Versprühen in einem heißen Luftstrom getrocknet wird. Die pharmazeutischen Wirkstoffe werden hierbei zumeist in Anwesenheit von Hilfsstoffen versprüht, die zum einen die Stabilität der Wirkstoffe erhalten sollen und zum anderen die Eigenschaften der sprühgetrockneten Pulver verbessern sollen.

Ein entscheidender Faktor bei der Stabilisierung durch Sprühtrocknung ist die Immobilisierung des Wirkstoffs in einer amorphen Matrix. Der amorphe Zustand besitzt eine hohe Viskosität mit geringer molekularer Beweglichkeit und geringer Reaktivität. Die Glasübergangstemperatur eines sprühgetrockneten Pulvers stellt hierbei einen wichtigen Parameter dar, da diese den Temperaturbereich angibt, bei dem der Übergang vom stabilen, amorphen in den weniger stabilen gummiartigen Zustand erfolgt. Vorteilhafte Hilfsstoffe müssen in der Lage sein eine amorphe Matrix mit möglichst hoher Glasübergangstemperatur zu bilden, in die der Wirkstoff eingebettet wird. Substanzen mit einer niedrigen Glasübergangstemperatur können bereits bei geringeren Temperaturen zerfließen und führen zu instabilen Pulverformulierungen. Die Auswahl der Hilfsstoffe hängt somit insbesondere von ihren Stabilisierungsfähigkeiten ab. Darüber hinaus spielen aber auch Faktoren wie die pharmazeutische Akzeptanz des Hilfsstoffs sowie dessen Einfluss auf die Teilchenbildung, die Dispergierbarkeit und die Fließeigenschaft eine entscheidende Rolle.

Die Sprühtrocknung stellt ein geeignetes Verfahren dar die chemische und physikalische Stabilität von peptid-/proteinartigen pharmazeutischen Wirkstoffen zu erhöhen (Maa et al., 1998, Pharmaceutical Research, 15(5), 768-775). Besonders im Bereich der pulmonalen Therapie wird die Sprühtrocknung zur Herstellung von peptid-/proteinhaltigen pulverartigen Arzneistoffen verwendet (US 5,626,874; US 5,972,388; Broadhead et al., 1994, J. Pharm. Pharmacol., 46(6), 458-467). Die inhalative Applikation von Peptiden/Proteinen stellt mittlerweile eine Alternative zu traditionellen Applikationsformen bei der Behandlung von systemischen Erkrankungen dar, da inhalativ applizierte Arzneistoffe neben einer lokalen auch eine systemische Wirkung entfalten können (WO 99/07340). Voraussetzung ist, dass die mittlere Teilchengröße im Bereich von 1-10 *µ*m, vorzugsweise 1-7,5 *µ*m liegt, so dass die Partikel in tiefere Lungenabschnitte und somit in den Blutkreislauf gelangen können. Die DE-A-179 22 07 beschreibt beispielhaft die Herstellung von entsprechenden Sprühtrocknungspartikeln, die eine ausreichende Dispergierbarkeit bei ihrer medizinischen Anwendung (Inhalation) aufweisen. Mittlerweile sind eine Vielzahl an Verfahren zur Herstellung inhalierbarer Pulver beschrieben (WO 95/31479; WO 96/09814; WO 96/32096; WO 96/32149; WO 97/41833; WO 97/44013; WO 98/16205; WO 98/31346; WO 99/66903; WO 00/10541; WO 01/13893; Maa et al., 1998, *supra;* Vidgrén et al., 1987, Int. J. Pharmaceutics, 35, 139-144; Niven et al.,1994, Pharmaceutical Research, 11(8), 1101-1109).

Als Hilfsstoffe haben sich unter anderem Zucker und deren Alkohole wie beispielsweise Trehalose, Lactose, Saccharose oder Mannitol, sowie verschiedene Polymere als geeignet erwiesen (Maa et al., 1997, Pharm. Development and Technology, 2(3), 213-223; Maa et al., 1998, supra; Dissertation Adler, 1998, Universität Erlangen; Costantino, et al., 1998, J. Pharm. Sci., 87(11), 1406-1411). Die vorwiegend eingesetzten Hilfsstoffe haben allerdings verschiedene Nachteile. Der Zusatz von Trehalose und Mannitol beispielsweise verschlechtert die Fließeigenschaften von Sprühtrocknungsformulierungen (C. Bosquillon et al., 2001 Journal of Controlled Release, 70(3), 329-339). Mannitol neigt zudem bei einem Gehalt von mehr als 20 Gewichtsprozent zur Rekristallisation (Costantino et al., 1998, supra), wodurch stabilisierende Effekte dramatisch abnehmen. Lactose, ein häufig verwendeter Hilfsstoff, verbessert zwar die Fließeigenschaften von Sprühtrocknungsformulierungen (C. Bosquillon et al., 2001, supra), ist aber insbesondere bei der Formulierung von peptid-/proteinhaltigen Wirkstoffen problematisch, da Lactose aufgrund ihrer reduzierenden Eigenschaft destabilisierende Maillard-Reaktionen mit Peptiden/Proteinen eingehen kann.

Dextrane mit einem Molekulargewicht von 40 bis 512 kDa finden vorwiegend bei der Gefriertrocknung von peptid-/proteinhaltigen Wirkstoffen Anwendung. Sie weisen einen amorphen Charakter mit gleichzeitig hohem Glasübergang auf. Diese hochmolekularen Dextrane sind jedoch aufgrund ihres starren Gerüstes nur bedingt in der Lage mit Peptiden/Proteinen adäquate Wasserstoffbrückenbindungen einzugehen und somit für eine hinreichende Stabilisierung während der Gefriertrocknung zu sorgen. Um diesen Nachteil auszugleichen werden sie u.a. mit Disacchariden kombiniert (Allison et al., 2000, J. Pharm. Sci., 89(2),199-214). Ein weiterer Nachteil hochmolekularer Dextrane besteht in ihrem hohen allergenen Potential (DextranAnapyhlaxie).

Liao et al, "Effects of sucrose and trehalose on the preservation of the native structure of spray-dried lysozyme", Pharmaceutical Research, bd.19, Nr.12, 2002 beschreibt sprühgetrocknete Pulver enthaltend Dextran und diskutiert die Stabilisierung von Wirkstoffen in sprühgetrockneten Pulvern. Insbesondere die Stabilität von sprühgetrocknetem Pulver Lysozym in Abhängigkeit von verschiedenen Zuckern bzw. Dextran als Hilfsstoff. Allerdings werden in dieser Arbeit keine Zusammensetzungen mit Isoleucin-haltigen Tripeptiden offenbart.

Eine Aufgabe der Erfindung bestand darin, neue Hilfsstoffe für die Herstellung von pulverartigen pharmazeutischen Zubereitungen zu Verfügung zu stellen. Die entsprechenden pulverartigen Zubereitungen sollten sich u.a. durch eine gute Langzeitstabilität und wenn möglich, durch Inhalierbarkeit auszeichnen.

Eine weitere Aufgabe der vorliegenden Erfindung bestand darin neue Hilfsstoffe für die Herstellung von sprühgetrockneten pharmazeutischen Zubereitungen bereit zu stellen. Die entsprechenden pulverartigen pharmazeutischen Zubereitungen sollten sich wiederum durch eine gute Langzeitstabilität und wenn möglich, durch Inhalierbarkeit auszeichnen.

Eine weitere Aufgabe der vorliegenden Erfindung bestand darin neue Hilfsstoffe für die Herstellung von peptid/-proteinhaltigen pharmazeutischen Formulierungen, insbesondere für solche die durch Sprühtrocknung entstehen, bereit zu stellen. Die entsprechenden peptid/-proteinhaltigen pharmazeutischen Zubereitungen sollten sich wiederum durch eine gute Langzeitstabilität und wenn möglich, durch Inhalierbarkeit auszeichnen.

Eine weitere Aufgabe der vorliegenden Erfindung bestand darin entsprechende pharmazeutische Zubereitungen für die inhalative Applikation bereit zu stellen, sei es in Form eines trockenen Pulvers, eines treibgashaltigen Dosieraerosols oder einer treibgasfreien Inhalationslösung.

Die der Erfindung zugrunde liegenden Aufgaben werden durch die nachfolgenden Ausführungen sowie durch die in den Patentansprüchen dargestellten Gegenstände/Verfahren gelöst.

### Zusammenfassung der Erfindung

Die vorliegende Erfindung betrifft sprühgetrocknetes Pulver enthaltend einen pharmazeutischen Wirkstoff, ein Tri-Isoleucin und ein niedermolekulares Dextran mit einem Molekulargewicht zwischen 500 und 10.000 Da. vorzugsweise zwischen ca. 500 und 5.000 Da, und besonders bevorzugt zwischen ca. 500 und 1.500 Da. Weitere erfindungsgemäße Ausführungsformen sind in den Unteransprüchen aufgeführt. Überraschenderweise wurde gefunden, dass die entsprechenden Pulver, nach ihrer Sprühtrocknung i) eine amorphe Struktur bilden, ii) in einer relativ hohe Ausbeute (zumindest 75% bezogen auf den eingesetzten Feststoff) resultieren, iii) über eine sehr hohe Glasübergangstemperatur verfügen (bis zu 65°C) und iv) über eine geringe Tendenz zur Rekristallisation verfügen. Als ein weiterer wichtiger Vorteil gegenüber z.B. sprühgetrockneter Trehalose weisen entsprechende sprühgetrocknete Pulver, die niedermolekulares Dextran enthalten, vorbesserte Fließeigenschaften auf.

Ein weiterer Vorteil gegenüber den im Stand der Technik beschriebenen pulverförmigen pharmazeutischen Zubereitungen, insbesondere gegenüber bekannten pulverförmigen sprühgetrockneten pharmazeutischen Zubereitungen, liegt in der besonders vorteilhaften Prozess- und Lagerstabilität der hier beschriebenen erfindungsgemäßen dextranhaltigen Pulver.

Der Anteil an niedermolekularem Dextran beträgt vorzugsweise in Bezug auf die Trockenmasse des Pulvers zwischen 50 und 99,99 % Gewichtsprozent (w/w), nach einer weiter bevorzugten Ausführungsform zwischen 55 und 99,99 % (w/w), vorzugsweise zwischen 60 und 99,99% (w/w).

Bei dem pharmazeutischen Wirkstoff handelt es vorzugsweise um ein biologisches Makromolekül, welches ein Polypeptid oder ein Protein, beispielsweise ein Wachstumsfakor, Enzym oder Antikörper sein kann. Besonders erfindungsgemäß sind demnach sprühgetrocknete Pulver (a) mit einem Anteil von 50 bis 99,99%, vorzugsweise 60 bis 99,99% (w/w) an niedermolekularem Dextran (bezogen auf die Trockenmasse des Pulvers) und (b) mit einem biologischen Makromolekül als pharmazeutischen Wirkstoff, vorzugsweise in einer Konzentration zwischen 0,01 und 40% (w/w), wiederum bezogen auf die Trockenmasse des Pulvers, wobei die Summe der Gewichtsprozente aus niedermolekularem Dextran und biologischem Makromolekül maximal 100 % (w/w) beträgt.

Die vorliegende Erfindung betrifft insbesondere sprühgetrocknete Pulver, welches in Bezug auf ihre Trockenmasse (a) ungefähr 60 bis 98,99% (w/w) eines niedermolekularen Dextrans, (b) ungefähr 1 bis 20% (w/w) eines Tri-Isoleucin und (c) ungefähr 0,01 bis 39% (w/w) eines pharmazeutischen Wirkstoffs, vorzugsweise eines Peptids/Proteins, beispielsweise eines Antikörpers, enthält.

Nach einer weiteren Ausführungsform betrifft die vorliegende Erfindung pharmazeutische Zusammensetzungen für inhalative Applikationen, die eines der hier beschriebenen erfindungsgemäßen Pulver enthalten oder aus diesen bestehen. In diesem Zusammenhang bevorzugt sind pharmazeutische Zusammensetzungen, die die erfindungsgemäßen Pulver als treibgashaltige Dosieraerosole oder als treibgasfreie Inhalationslösungen enthalten. Die zur Herstellung der pharmazeutischen Zusammensetzung verwendeten erfindungsgemäßen sprühgetrockneten Pulver zeichnen sich nach einer weiteren Ausführungsform durch einen hohen Anteil an inhalierbaren Teilchen mit einem mittleren aerodynamischen Teilchendurchmesser
(MMAD) von kleiner 10 *µ*m, vorzugsweise von 0,5 - 7,5 *µ*m, weiter bevorzugt vom 0,5 - 5,5 *µ*m, besonders bevorzugt von 0,5 - 5,0 *µ*m aus.

Die Erfindung stellt ferner Verfahren zur Herstellung der entsprechenden erfindungsgemäßen sprühgetrockneten Pulver zur Verfügung, dadurch gekennzeichnet, dass eine Lösung oder Suspension, die zumindest ein niedermolekulares Dextran und einen pharmazeutischen Wirkstoff enthält, hergestellt wird und diese unter geeigneten Bedingungen versprüht wird. Die Temperatur für den Sprühprozess liegt vorzugsweise zwischen 50 und 200°C (Einströmtemperatur) und 30 und 150°C (Ausströmtemperatur).

### Beschreibung der Abbildungen

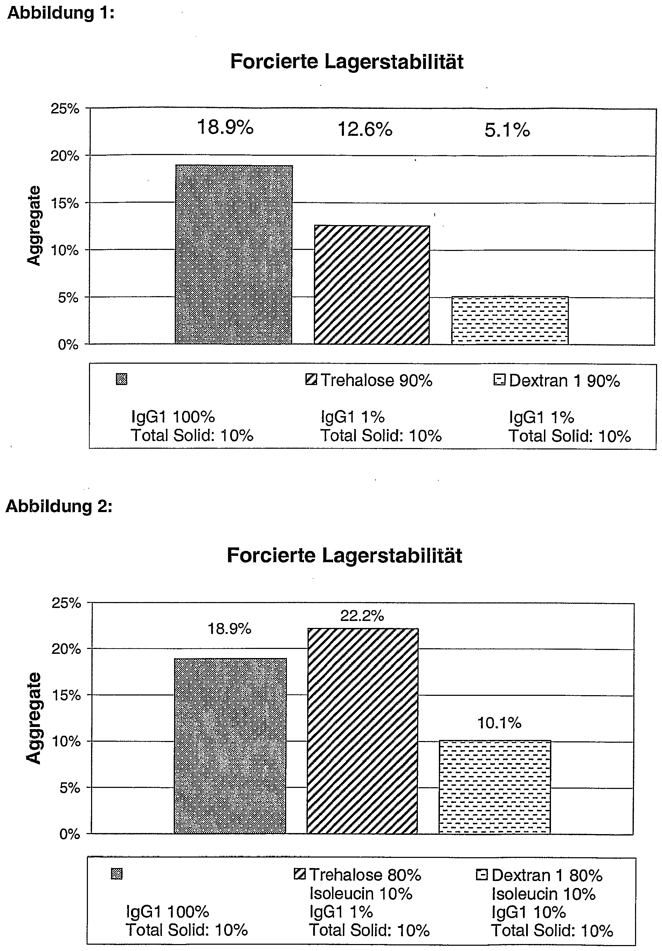

**Abbildung 1** zeigt die Aggregatbildung nach Sprühtrocknung, forcierter Lagerung und Rekonstitution. Versprüht wurden wässrige Lösungen mit a) 10% (w/w) IgG-Anteil, b) 1% (w/w) IgG- und 9% Trehalose-Anteil und c) 1% (w/w) IgG- und 9% Dextran₁₀₀₀-Anteil. Das dextranhaltige Pulver zeichnet sich durch einen geringen Anteil an Aggregaten aus.

**Abbildung 2** zeigt die Aggregatbildung nach Sprühtrocknung, forcierter Lagerung und Rekonstitution. Versprüht wurden wässrige Lösungen mit a) 10% (w/w) IgG-Anteil, b) 1% (w/w) IgG, 1% (w/w) Isoleucin- und 8% Trehalose-Anteil und c) 1% (w/w) IgG-, 1% (w/w) Isoleucin- und 8% (w/w) Dextran₁₀₀₀-Anteil aus. Das dextranhaltige Pulver zeichnet sich durch einen geringen Anteil an Aggregaten aus.

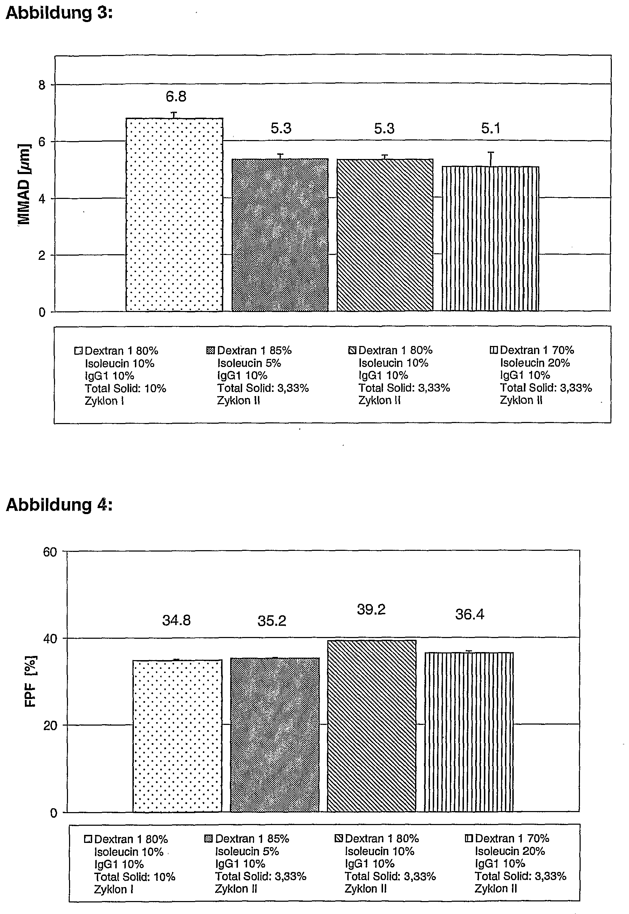

**Abbildung 3** zeigt die Mass Mean Aerodynamic Diameter (MMAD) verschiedener Pulver, welche durch Sprühtrocknung wässriger Lösungen hergestellt wurden, die Dextran₁₀₀₀, Isoleucin und IgG enthalten. Die Lösungen wurden wie unter BEISPIELE beschrieben hergestellt und versprüht. Sämtliche Pulver haben einen MMAD von kleiner 7,5 *µ*m. Das Diagramm zeigt den Einfluss des Isoleucin-Anteils bei konstanten Gesamtfeststoffkonzentrationen und Sprühparametern auf den MMAD. Ein höherer Isoleucin-Anteil an der Formulierung setzt den MMAD herab. Total Solid: Feststoffanteil in der Sprühlösung. Zyklon I: Büchi Cyclone. Zyklon II: Büchi High-performance Cyclone.

**Abbildung 4** zeigt die Fine Particle Fraction (FPF) mit einem Cut Off Diameter kleiner 5 *µ*m für verschiedene Pulver, welche durch Sprühtrocknung wässriger Lösungen hergestellt wurden, die Dextran₁₀₀₀, Isoleucin und IgG enthalten. Die Lösungen wurden wie unter BEISPIELE beschrieben hergestellt und versprüht. Sämtliche Pulver haben eine FPF größer 30 %, oder auch von größer 35 %. Total Solid: Feststoffanteil in der Sprühlösung. Zyklon I: Büchi Cyclone . Zyklon II: Büchi High-performance Cyclone.

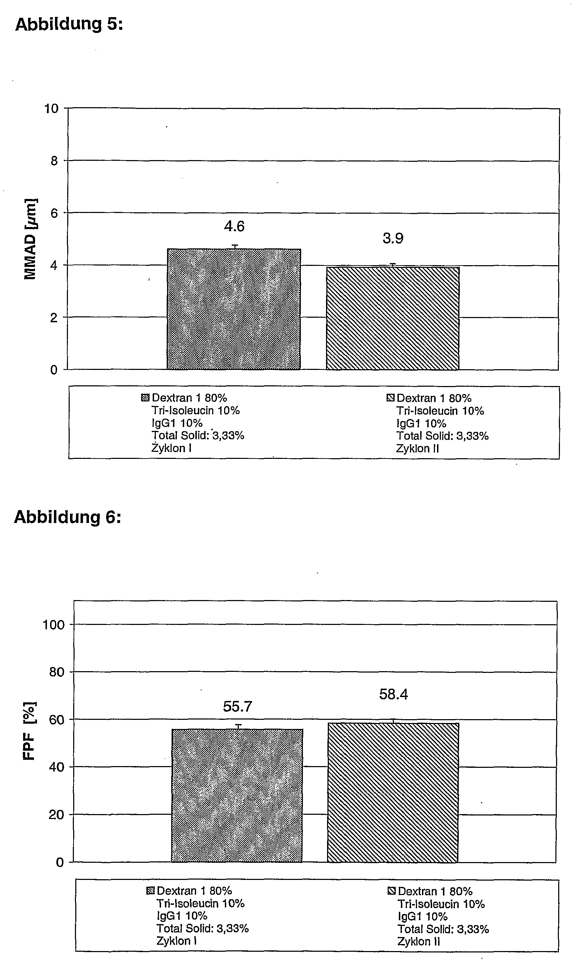

**Abbildung 5** zeigt die Mass Mean Aerodynamic Diameter (MMAD) verschiedener Pulver, welche durch Sprühtrocknung wässriger Lösungen hergestellt wurden, die Dextran₁₀₀₀, Tri-Isoleucin und IgG enthalten. Die Lösungen wurden wie unter BEISPIELE beschrieben hergestellt und versprüht. Beide Pulver haben einen MMAD kleiner 5 *µ*m, bzw. auch kleiner 4 *µ*m. Total Solid: Feststoffanteil in der Sprühlösung. Büchi Cyclone. Zyklon II: Büchi High-performance Cyclone.

**Abbildung 6** zeigt die Fine Particle Fraction (FPF) mit einem Cut Off Diameter kleiner 5 *µ*m von verschiedenen Pulvern, welche durch Sprühtrocknung wässriger Lösungen hergestellt wurden, die Dextran₁₀₀₀, Tri-Isoleucin und IgG enthalten. Die Lösungen wurden wie unter BEISPIELE beschrieben hergestellt und versprüht. Beide Pulver haben eine FPF von größer 55%, bzw. 58%. Total Solid: Feststoffanteil in der Sprühlösung. Büchi Cyclone. Zyklon II: Büchi High-performance Cyclone.

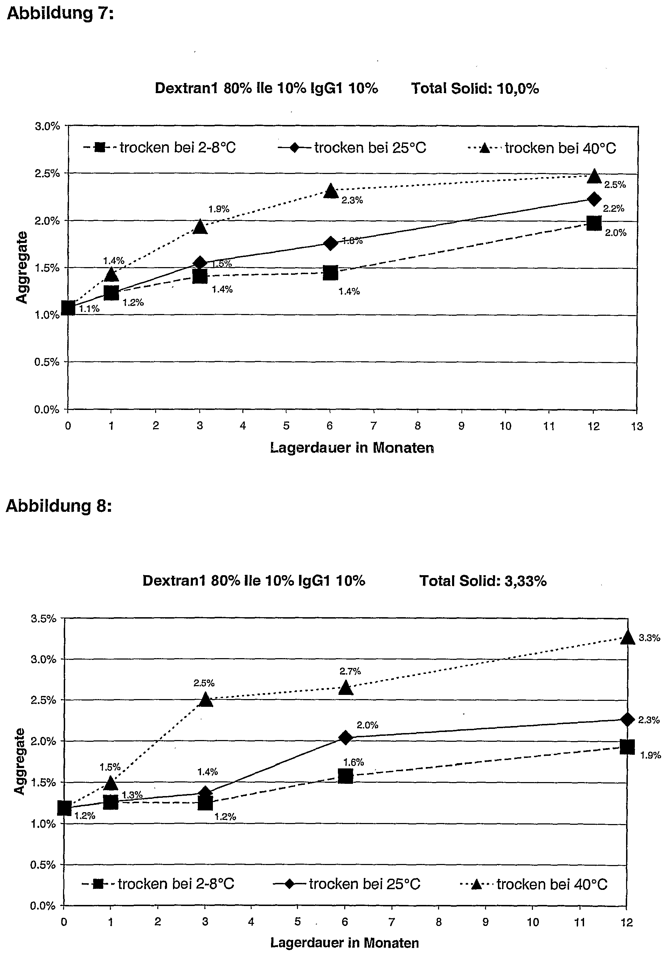

**Abbildung 7** zeigt die Aggregatbildung nach Sprühtrocknung, bis zu einjähriger Lagerung bei 2-8, 25 und 40°C und nachfolgender Rekonstitution. Versprüht wurde eine wässrige Lösung mit 1% (w/w) IgG-Anteil, 1% (w/w) Isoleucin- 8% (w/w) Dextran₁₀₀₀-Anteil (siehe Beispiel 2). Das dextranhaltige Pulver zeichnet sich jeweils nach 3, 6, 9 und 12 monatiger Lagerung bei 2-8°C, 25°C, 40°C durch einen geringen Anteil an Aggregaten aus.

**Abbildung 8** zeigt die Aggregatbildung nach Sprühtrocknung, bis zu einjähriger Lagerung bei 2-8, 25 und 40°C und nachfolgender Rekonstitution. Versprüht wurde eine wässrige Lösung mit 0,33% (w/w) IgG-Anteil, 0,33% (w/w) Isoleucin- 2,66% (w/w) Dextran-₁₀₀₀-Anteil (siehe Beispiel 2). Das dextranhaltige Pulver zeichnet sich nach einjähriger Lagerung bei 2-8°C, 25°C, 40°C durch einen geringen Anteil an Aggregaten aus.

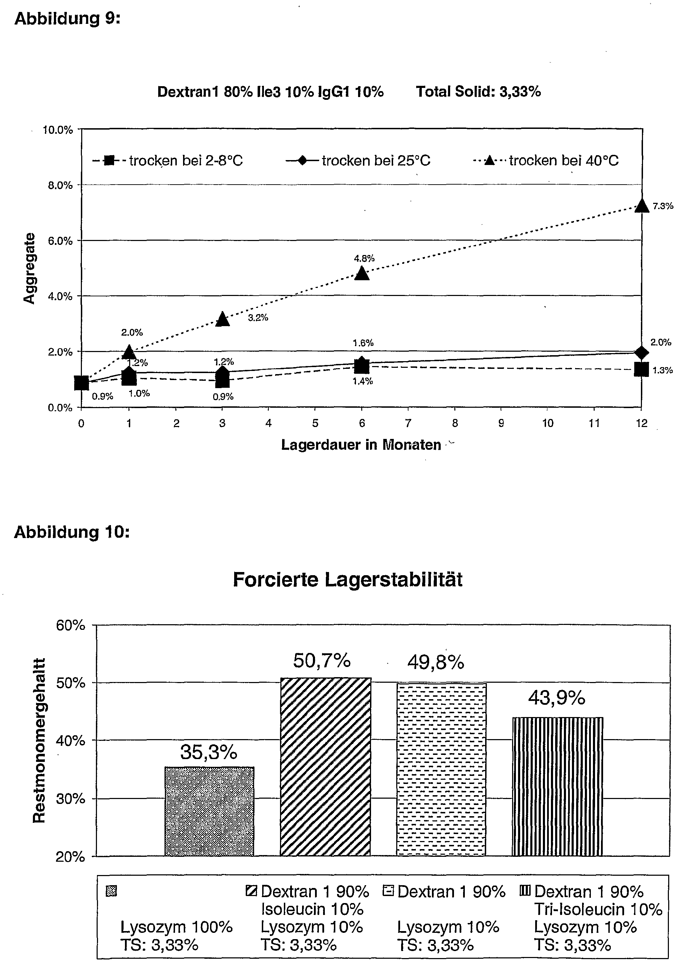

**Abbildung 9** zeigt die Aggregatbildung nach Sprühtrocknung, bis zu einjähriger Lagerung bei 2-8, 25 und 40°C und nachfolgender Rekonstitution. Versprüht wurde eine wässrige Lösung mit 0,33% (w/w) IgG-Anteil, 0,33% (w/w) Tri-Isoleucin- 2,66% (w/w) Dextran₁₀₀₀-Anteil (siehe Beispiel 3). Das dextranhaltige Pulver zeichnet sich nach einjähriger Lagerung bei 2-8°C, 25°C, 40°C durch einen geringen Anteil an Aggregaten aus.

**Abbildung 10** zeigt den Restmonomergehalt nach Sprühtocknung, forcierter Lagerung und Rekonstitution. Versprüht wurden wässrige Lösungen mit a) 3,33% (w/w) Lysozym-Anteil, b) 0,33% (w/w) Lysozym- und 3,0% Dextran₁₀₀₀-Anteil, c) 0,33% (w/w) Lysozym-, 0,33% (w/w) Isoleucin- und 2,66% (w/w) Dextran₁₀₀₀-Anteil sowie d) 0,33% (w/w) Lysozym-, 0,33% (w/w) Tri-Isoleucin- und 2,66% (w/w) Dextran₁₀₀₀-Anteil. Das dextranhaltige Pulver zeichnet sich durch hohen Restmonomergehalt aus.

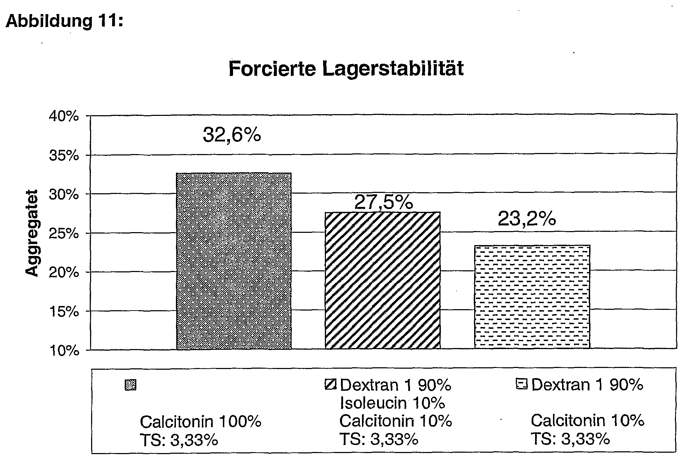

**Abbildung 11** zeigt den Aggregatgehalt nach Sprühtrocknung, forcierter Lagerung und Rekonstitution. Versprüht wurden wässrige Lösungen mit a) 3,33% (w/w) Calcitonin-Anteil, b) 0,33% (w/w) Calcitonin- und 3,0% Dextran₁₀₀₀-Anteil sowie c) 0,33% (w/w) Calcitonin-, 0,33% (w/w) Isoleucin- und 2,66% (w/w) Dextran₁₀₀₀-Anteil. Das dextranhaltige Pulver zeichnet sich durch geringen Aggregatgehalt aus.

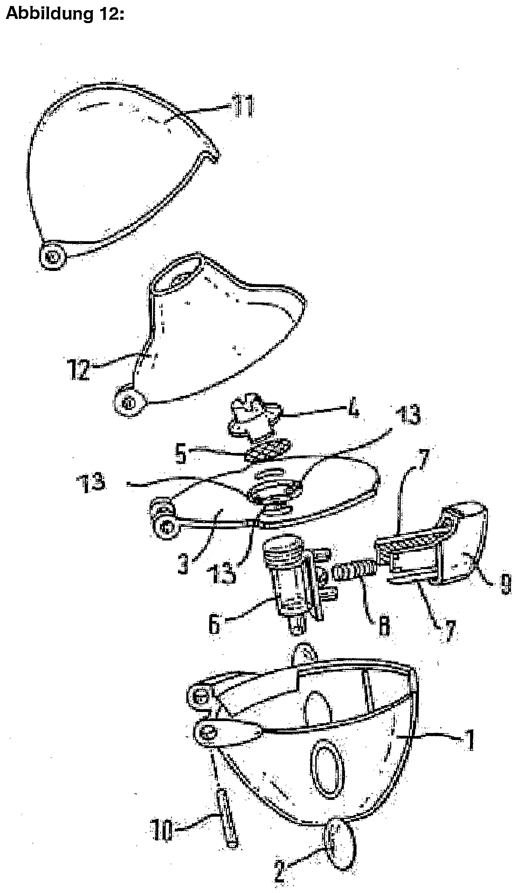

**Abbildung 12** zeigt einen Inhalator zur inhalativen Applikation von trockenen Pulverzubereitungen.

### Detaillierte Beschreibung der Erfindung

### Definitionen

Im Rahmen dieser Erfindungsbeschreibung verwendete Begriffe und Bezeichnungen haben folgende im Anschluss definierte Bedeutungen. Die Gewichts- und Gewichtsprozentangaben beziehen sich, sofern nichts anderes erwähnt wird, jeweils auf die Trockenmasse der Pulver oder den Feststoffgehalt der zu versprühenden Lösungen / Suspensionen.

Der Ausdruck "Dextran 1" oder "Dextran ₁₀₀₀" meint ein niedermolekulares Dextran mit einem mittleren Molekulargewicht von ca. 1.000 Dalton. Die in dieser Patentschrift angebenen Molekulargewichte für Dextran beziehen sich immer auf das mittlere Molekulargewicht. Dies meint das die verwendeten Dextrane zumeist polymorph sind. Das mittlere Molekulargewicht gibt hierbei an, das zumindest 50%, vorzugsweise 60%, weiter bevorzugt 70%, noch weiter bevorzugt 80%, noch weiter bevorzugt 90%, noch weiter bevorzugt 92% , noch weiter bevorzugt 94%, noch weiter bevorzugt 96%, noch weiter bevorzugt 98% und noch weiter bevorzugt 99% der Dextrane ein dem Zahlenwert entsprechendes Molekulargewicht aufweisen.

Mit dem Ausdruck "sprühgetrocknete Pulverformulierung" oder "trockene Pulverformulierung" sind Pulverformulierungen gemeint, welche üblicherweise weniger als ca. 10% (w/w) Restfeuchte, vorzugsweise weniger als 7% (w/w) Restfeuchte, besonders bevorzugt weniger als 3% (w/w) Restfeuchte und noch weiter bevorzugt weniger als 2% (w/w) Restfeuchte aufweisen. Die Restfeuchte ist im wesentlichen von Art und Anteil des pharmazeutischen Wirkstoffs in der Pulverformulierung abhängig.

Der Begriff "amorph" meint, dass die pulverförmigen Formulierung weniger als 10% kristalline Anteile enthalten, vorzugsweise weniger als 7%, weiter bevorzugt weniger als 5%, insbesondere weniger als 4, 3, 2, oder 1%.

Der Begriff "inhalierbar" meint, dass die Pulver zur pulmonalen Applikation geeignet sind. Inhalierbare Pulver lassen sich mit Hilfe eines Inhalationsgerätes dispergieren und inhalieren, so dass die Partikel die Lunge erreichen und gegebenenfalls über die Alveolen systemische Wirkung entfalten können. Inhalierbare Partikel weisen beispielsweise eine mittlere Teilchengröße zwischen 0,4-10 *µ*m (MMD = Mass median diameter), meistens zwischen 0,5-5 *µ*m, bevorzugt zwischen 1-3 *µ*m und/oder einen mittleren aerodynamischen Teilchendurchmesser (MMAD = Mass median aerodynamic diameter) zwischen 0,5-10 *µ*m, vorzugsweise zwischen 0,5-7,5 *µ*m, weiter bevorzugt zwischen 0,5-5,5 *µ*m, noch weiter bevorzugt 1-5 *µ*m und in besonders bevorzugter Weise zwischen 1-4,5 *µ*m auf.

"Mass Median Diameter" oder "MMD" ist eine Messgröße für die durchschnittliche Partikelgrößenverteilung, da die Pulver der Erfindung generell polydispers sind. Die Ergebnisse werden ausgedrückt als Durchmesser der Volumensummenverteilung bei 50% Durchgangssumme. Die MMD-Werte lassen sich beispielhaft mittels Laserdiffraktometrie (vgl. hierzu: Kapitel BEISPIELE, Methode) bestimmen, wobei natürlich auch jede andere übliche Methode verwendet werden kann (z.B. Elektronenmikroskopie, Zentrifugalsedimentation).

Der Begriff "mittlerer aerodynamischer Teilchendurchmesser" (= mass median aerodynamic diameter (MMAD)) gibt die aerodynamische Teilchengröße an, bei der normalerweise 50% der Teilchen des Pulvers einen kleineren aerodynamischen Durchmesser aufweisen. Als Referenzmethode zur Bestimmung des MMAD dient im Zweifel die in dieser Patentschrift angegebene Methode (vgl. hierzu: Kapitel BEISPIELE, Methode).

Der Begriff *"Fine Particle Fraction"* (FPF) beschreibt den inhalierbaren Teil eines Pulvers bestehend aus Teilchen mit einer Teilchengröße von ≤ 5 *µ*m MMAD. In gut dispergierbaren Pulvern beträgt die FPF mehr als 20%, vorzugsweise mehr als 30%, besonders bevorzugt mehr als 40%, noch weiter bevorzugt mehr als 50%, noch weiter bevorzugt mehr als 55%. Der in diesem Zusammenhang verwendete Begriff "Cut Off Diamenter" gibt an, welche Partikel bei der Bestimmung der FPF berücksichtigt werden. Eine FPF von 30% bei einem Cut Off Diameter von 5 *µ*m (FPF₅) meint, dass zumindest 30% aller Partikel im Pulver einen mittleren aerodynamischen Teilchendurchmesser von kleiner 5 *µ*m aufweisen.

Der Begriff "Sprühlösung" meint wässrige Lösungen oder Suspensionen, in denen der pharmazeutische Wirkstoff zusammen mit zumindest einem Hilfsstoff gelöst / suspendiert ist.

Der Begriff "time of flight" ist die Bezeichnung für eine Standardmessmethode wie im Kapitel BEISPIELE näher beschrieben. Bei einer time of flight Messung werden simultan der MMAD und FPF bestimmt (vgl. hierzu: Kapitel BEISPIELE, Methode).

Der Begriff "pharmazeutisch akzeptable Hilfsstoffe", "Trägermaterial" oder "Matrizes" bezieht sich auf Hilfsstoffe, die optional im Rahmen der Erfindung in der Formulierung enthalten sein können. Die Hilfsstoffe können beispielsweise pulmonal appliziert werden ohne hierbei signifikant ungünstige toxikologische Effekte auf den Probanden oder die Probandenlunge zu haben.

Der Ausdruck "pharmazeutisch akzeptable Salze" umfasst beispielsweise folgende Salze, ist aber nicht begrenzt auf diese: Salze aus anorganischen Säuren, wie Chlorid, Sulfat, Phosphat, Diphosphat, Bromid und Nitratsalze. Des weiteren Salze aus organischen Säuren, wie Malat, Maleat, Fumarat, Tartrat, Succinat, Etyhlsuccinat, Citrat, Acetat, Lactat, Methansulfonat, Benzoat, Ascorbat, Para-Toluolsulfonat, Palmoat, Salicylat und Stearat, ebenso wie Estolat, Gluceptat und Lactobianat Salze.

Der Begriff "pharmazeutisch akzeptable Kationen" umfasst, ohne auf diese begrenzt zu sein, beispielsweise Lithium, Natrium, Kalium, Calcium, Aluminium sowie Ammonium (inklusive substituiertem Ammonium).

Unter einem "pharmazeutischen Wirkstoff" ist eine Substanz, ein Arzneimittel, eine Zusammensetzung oder eine Kombination hieraus zu verstehen, die einen pharmakologischen, zumeist positiven, Effekt auf einen Organismus, ein Organ, und/oder eine Zelle ausübt, wenn der Wirkstoff mit dem Organismus, Organ oder der Zelle in Kontakt gebracht wird. Eingebracht in einen Patienten, kann der Effekt lokal oder systemisch sein.

Der Begriff "biologisches Makromolekül" meint Peptide, Proteine, Fette, Fettsäuren, oder auch Nukleinsäuren.

Mit dem Begriff "Peptid" oder "Polypeptid" sind Polymere von Aminosäuren bestehend aus zwei bis hundert Aminosäureresten gemeint. Der Begriff Peptid oder Polypeptid wird als pseudonym verwendet und umfasst sowohl Homo- als auch Heteropeptide, also Polymere von Aminosäuren bestehend aus identischen oder verschiedenen Aminosäureresten. Ein "Di-Peptid" ist somit aus zwei peptidisch verknüpften Aminosäuren, ein "Tri-Peptid" aus drei peptidisch verknüpften Aminosäuren aufgebaut. Der hier verwendete Begriff "Protein" meint Polymere von Aminosäuren mit mehr als 100 Aminosäureresten.

Der Begriff "Analoge" bezeichnet Peptide/Proteine in denen einzelnen oder mehrere Aminosäuren substituiert, eliminiert (z.B. Fragmente), addiert (z.B. Derivate mit einer C- oder N-terminalen Verlängerung) oder anderweitig von der nativen (Wildtyp) Sequenz modifiziert wurden. Ebenso ist auch eine Derivatisierung des nativen Proteins, z.B. durch Zucker, Polyethylenglykol o. a. möglich. Analoge haben eine Bioaktivität von zumindest 10, 20, 30 oder 40%, bevorzugt von zumindest 50, 60 oder 70% und besonders bevorzugt von zumindest 80, 90, 95 100% oder mehr als 100% Bioaktivität des nativen, nicht synthetischen Proteins.

Der Ausdruck "Aminosäure" meint Verbindungen, welche mindestens eine Amino- und mindestens eine Carboxylgruppe enthalten. Obwohl die Aminogruppe üblicherweise in α-Position zur Carboxylgruppe steht ist auch jede andere Anordnung im Molekül denkbar. Die Aminoäure kann auch weitere funktionelle Gruppen, wie z.B. Amino-, Carboxamid-, Carboxyl-, Imidazol-, Thiogruppen und andere Gruppen, enthalten. Verwendung finden Aminosäuren natürlichen oder synthetischen Ursprungs, razemisch oder optisch aktiv (D-, oder L-) inklusive verschiedener stereoisomerer Verhältnisse. Beispielsweise umfasst der Begriff Isoleucin sowohl D- Isoleucin, L-Isoleucin, razemisches Isoleucin und verschiedene Verhältnisse der beiden Enantiomere.

Der Ausdruck "pure oder reine Proteinformulierung" meint sprühgetrocknete Pulver bestehend aus einem oder mehreren Proteinen und optional einem geeigneten Puffer (typischerweise von 0 bis 15% (w/w) bezogen auf das Gewicht des trockenen Pulvers). Das Pulver enthält grundsätzlich keine weiteren Hilfsstoffe,_ d.h. der Gehalt an eventuellen weiteren Hilfsstoffen ist weniger als 1% (w/w) bezogen auf das Gewicht des trockenen Pulvers.

Eine "oberflächenaktive" Substanz ist in der Lage die Oberflächenspannung der Lösung, in welcher sie gelöst ist, herabzusetzen. Die Oberflächenaktivität wird beispielsweise durch die Tensiometer - Methode nach Lecomte du Noüy (Bauer, Frömming, Führer, 6. Auflage) gemessen.

### Erfindungsgemäße Pulver

Die vorliegende Erfindung betrifft Sprühgetrocknetes Pulver enthaltend einen pharmazeutischen Wirkstoff, ein Tri-Isoleucin und ein niedermolekulares Dextran mit einem Molekulargewicht zwischen 500 und 10.000Da Im weiteren Verlauf werden diese neuen und überraschenderweise überlegen Sprühgetrockneten Pulver näher beschrieben und charakterisiert.

Dextran ist üblicherweise ein hochmolekulares Glukosepolymer. Es kann beispielsweise durch Kultivierung von Leuconostoc Mesenteroides B512F in Gegenwart von Saccharose hergestellt werden. Natives Dextran kann durch partielle saure Hydrolyse nach entsprechenden Aufreinigungsschritten in gewünschte Molekulargewichtsfraktionen gewonnen werden. Dextran ist ein (1->6) verknüpftes a-D-Glucan mit an O-3-Positon gebunden Seitenketten. Das Ausmaß der Verzweigungen beträgt meistens ca. 5%. Die Verzweigungen sind meisten 1-2 Glukoseeinheiten lang. Die üblicherweise verwendeten Dextrane haben mittlere Molekulargewichte deutlich größer als 10.000 Da. (meisten 40.000, 70.000 oder 512.000 Da). Das im erfindungsgemäßen sprühgetrockneten Pulver enthaltene Dextran dagegen hat lediglich ein mittleres Molekulargewicht von bis zu 10.000 Da, vorzugsweise von bis zu 5.000 Da, besonders bevorzugt von bis zu 1.500 Da. Im Rahmen der vorliegenden Erfindung hat sich gezeigt, dass sich Dextran mit einem mittleren Molekulargewicht von ungefähr 1.000 Da. besonders gut als Stabilisator bei der Herstellung von partikulären Pulvern eignet.

Vorteile der pharmazeutischen Dextrane:
- USP und Ph. Eur. Monographiert
- frei von Leuconostoc Antigen
- zugelassen für i.v.-Applikation
- vollständig biologisch abbaubar zu Kohlendioxid und Wasser
- stabil bei Raumtemperatur für 5 Jahre
spezieller Vorteil niedermolekularen Dextran (-1.000 Dalton)
- geringe Antigenizität
Insgesamt sollte der Anteil an niedermolekularem Dextran so gewählt werden, dass das sprühgetrocknete Pulver zumindest teilamorph, vorzugsweise vollständig amorph ist. Der Anteil an niedermolekularen Dextran kann auch unter 50% (w/w) abgesenkt werden, sofern dem Pulver weitere stabilisierende Hilfsstoffe in geeigneter Menge zugesetzt werden. Beispiele für weitere stabilisierende Hilfsstoffe finden sich in dieser Patentschrift an anderer Stelle.

Der Anteil an pharmazeutischen Wirkstoff an der Trockenmasse der erfindungsgemäßen Pulver beträgt in der Regel zwischen 0,01 und 50% (w/w), vorzugsweise zwischen 0,33 und 50% (w/w), weiter bevorzugt zwischen 0,33 und 45% (w/w), noch weiter bevorzugt zwischen 0,33 und 40% (w/w). Nach einer weiter bevorzugten Ausführungsform beträgt der Anteil des pharmazeutischen Wirkstoffs am Feststoffgehalt des erfindungsgemäßen Pulvers zwischen 0,33 und 35% (w/w), vorzugsweise zwischen 0,33 und 30% (w/w), weiter bevorzugt zwischen 0,33 und 25% (w/w) und noch weiter bevorzugt zwischen 0,33 und 10% (w/w). Der Anteil beträgt somit beispielsweise 0,01, 0,02, 0,03 ... 0,08, 0,09, etc.; 0,1, 0,2 0,3, ... 0,8, 0,9 etc.; 1, 2, 3, ... 8 ,9, 10 etc.; 11, 12, 13, ... 18, 19, 20 etc.; 21, 22, 23, ... 28 ,29, 30 etc.; 31, 32, 33, ... 38 ,39;40 etc.; 41, 42, 43, ... 48 ,49, etc; 49,1, 49,2, 49,3, ... 49,8, 49,9, etc.; 49,91, 49,92, 49,93, ... 49,98, 49,99 (w/w).

Erfindungsgemäß sind demnach Pulver mit einem Verhältnis von niedermolekularem Dextran zu Wirkstoff von beispielsweise 50/50, 51/49, 52/48, 53/47, 54/46, 55/45, 56/44, 57/43, 58/42, 59/41, 60/40, 61/39, 62/38, 63/37, 64/36, 65/35, 66/34, 67/33, 68/32, 69/31, 70/30, 71/29, 72/28, 73/27, 74/26, 75/25, 76/24, 77/23, 78/22, 79/21, 80/20, 81/19, 82/18, 83/17, 84/16, 85/15, 86/14, 87/13, 88/12, 89/11, 90/10, 91/9, 92/8, 93/7, 94/6, 95/5, 96/4, 97/3, 98/2, 99/1, 99,1/0,9, 99,2/0,8, 99,3/0,7, 99,4/0,6, 99,5/0,5, 99,6/0,4, 99,66/0,33, 99,7/0,3, 99,8/0,2, 99,9/0,1, 99,99/0,01 (w/w). Enthält das entsprechende Pulver einen oder mehrere zusätzliche Hilfsstoffe, so kann entweder der Anteil an niedermolekularem Dextran, der Anteil an pharmazeutischen Wirkstoff, oder beide Anteile entsprechend reduziert werden, wobei der Anteil an niedermolekularem Dextran bezogen auf die Trockenmasse des Pulvers vorzugsweise einen der Werte zwischen 50 und 99,99% (w/w) aufweist.

Pharmazeutische Wirkstoffe im Sinne der Erfindung sind, neben denen, die unter die allgemeine Definition fallen, unter anderem Antibiotika, anti-virale Wirkstoffe, Anepileptika, Schmerzmittel (analgesics), anti-inflammatorische Wirkstoffe oder Bronchodilatoren. Ferner zählen hierzu Wirkstoffe die beispielsweise auf das periphere Nervensystem, auf adrenergische Rezeptoren, cholinergische Rezeptoren, die skeletale Muskulatur, das cardiovaskuläre System, die glatte Muskulatur, das Blut zirkulierende System, auf synaptische Stellen, neuroeffektorische Verbindungsstellen, das endokrine System, das Immunsystem, das reproduktive System, das skeletale System, die autakoiden Systeme, die alimentarischen und excretorischen Systeme, das Histaminsystem und das zentrale Nervensystem wirken. Geeignete Wirkstoffe umfassen ferner beispielsweise. Hypnotika und Sedativa, psychische Energizer, Tranquilizer, anti-Convulsanten, Muskelrelaxanten, anti-parkinson Wirkstoffe, Schmerzmittel, anti-inflammatorische Wirkstoffe, Muskelkontraktanten, anti-mikrobielle Wirkstoffe, hormonale Wirkstoffe, wie beispielsweise Kontrazeptiva, Sympathomimetika, Diuretika, Fettstoffwechsel regulierende Wirkstoffe, antiandrogenische Wirkstoffe, Antiparasitika, Neoplastika, Antineoplastika und Hypoglycemika.

Ferner sind von dem Begriff pharmazeutischer Wirkstoff beispielhaft solche Wirkstoffe umfasst, die auf das respiratorische System, beispielsweise gegen eine der folgenden Krankenheiten wirken: Asthma, chronische obstruktive pulmonarische Erkrankungen (COPD), emphysemische chronische Bronchitis, bronchopulmonarische Dysplasie (BPD), neonatales respiratorisches Distress Syndrom (RDS), Bronchiolitis, Krupp, post-extubation Stridor, pulmonarische Fibrose, Pneumonie oder cystische Fibrose (CF).

Repräsentative Beispiele für Bronchodilatoren umfassen neben anderen beta-Agonisten, Anticholinergika oder Methylxanthine. Beispiele für anti-inflammatorische Wirkstoffe sind Steroide, Cromolyn, Nedokromil und leukotriene Inhibitoren. Beispiele für Steroide umfassen Beclomethason, Betamethason, Biclomethason, Dexamethason, Triamcinolon, Budesonid, Butixocort, Ciclesonid, Flutikason, Flunisolid, Icomethason, Mometason, Tixocortol, und loteprednol. Weitere Beispiele sind Budesonid, Fluticason propionate, Beclomethason dipropionat, Fometerol und Triamcinolon acetonid.

Beispiele für anti-mikrobiell wirkende Wirkstoffe sind Erythromycin, Oleandomycin, Troleandomycin, Roxithromycin, Clarithromycin, Davercin, Azithromycin, Flurithromycin, Dirithromycin, Josamycin, Spiromycin, Midecamycin, Leucomycin, Miocamycin, Rokitamycin, Andazithromycin und Swinolide A; Fluoroquinolone beispielsweise Ciprofloxacin, Ofloxacin, Levofloxacin, Trovafloxacin, Alatrofloxacin, Moxifloxicin, Norfloxacin, Eoxacin, Grepafloxacin, Gatifloxacin, Lomefloxacin, Sparfloxacin, Temafloxacin, Pefloxacin, Amifloxacin, Fleroxacin, Tosufloxacin, Prulifloxacin, Irloxacin, Pazufloxacin, Clinafloxacin und Sitafloxacin; Aminoglykoside wie zum Beispiel Gentamicin, Netilmicin, Paramecin, Tobramycin, Amikacin, Kanamycin, Neomycin; Streptomycin, Vancomycin, Teicoplanin, Rampolanin, Mideplanin, Colistin, Daptomycin, Gramicidin, Colistimethate; Polymixine wie zum Beispiel Polymixin B, Capreomycin, Bacitracin, Peneme, Penicilline einschließlich penicillinase-sensitive Wirkstoffe wie Penicillin G, Penicillin V, penicillinase-resistente Wirkstoffe wie Methicillin, Oxacillin, Cloxacillin, Dicloxacillin, Floxacillin, Nafcillin; aktive Wirkstoffe gegen Gram-negative Bakterien wie Ampicillin, Amoxicillin, Hetacillin, Cillin, und Galampicillin; anti-pseudomonale Penicilline wie Carbenicillin, Ticarcillin, Azlocillin, Mezlocillin, Andpiperacillin; Cephalosporine wie Cefpodoxim, Cefprozil, Ceftbuten, Ceftizoxime, Ceftriaxon, Cephalothin, Cephapirin, Cephalexin, Cephradrin, Cefoxitin, Cefamandol, Cefazolin, Cephaloridin, Cefaclor cefadroxil, Cephaloglycin, Cefuroxim, Ceforanid, Cefotaxim, Cefatrizin, Cephacetril, Cefepim, Cefixim, Cefonizid, Cefoperazon, Cefotetan, Cefmetazol, Ceftazidim, Loracarbef und Moxalactam; Monobaktame wie Aztreonam; und Carbapeneme wie zum Beispiel Imipenem, Meropenem pentamidin isethiouat, Aalbuterol sulfat, Lidocain, Metaproterenol sulfat, Beclomethason diprepionat, Triamcinolon acetamid, Budesonid acetonid, Fluticason, Ipratropium bromid, Flunisolid, Cromolyn Natrium, Ergotamin tartrat und wo anwendbar, Analoge, Agonisten, Antagonisten, Inhibitoren und pharmazeutische verwendbare Salzformen und der gleichen hiervon.

Bei dem pharmazeutischen Wirkstoff handelt es sich nach einer weiteren Ausführungsform um ein biologisches Makromolekül. Entsprechend der oben angegebenen Definition sind hierunter beispielsweise Peptide, Proteine, Fette, Fettsäuren, oder auch Nukleinsäuren zu verstehen.

Biopharmazeutisch bedeutsame Proteine/Polypeptide umfassen z.B. Antikörper, Enzyme, Wachstumsfaktoren z.B. Steroide, Cytokine, Lymphokine, Adhäsionsmoleküle, Rezeptoren sowie deren Derivate bzw. Fragmente, sind aber nicht auf diese beschränkt. Im Allgemeinen sind alle Polypeptide bedeutsam, die als Agonisten oder Antagonisten wirken und/oder therapeutische oder diagnostische Anwendung finden können.

Geeignete Peptide oder Proteine im Sinne der Erfindung sind beispielsweise Insulin, Insulin-ähnlicher Wachstumsfaktor, humanes Wachstumshormon (hGH) und andere Wachstumsfaktoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, Interferon (IFN)-alpha, beta, gamma, omega oder tau, Tumornekrosefaktor (TNF) wie z.B. TNF-alpha, beta oder gamma, TRAIL, G-CSF, GM-CSF, M-CSF, MCP-1 und VEGF. Weitere Beispiele sind monoklonale, polyklonale, multispezifische und einzelkettige (*single chain*) Antikörper und Fragmente davon, wie z.B. Fab, Fab', F(ab')₂, Fc und Fc'-Fragmente, leichte (L) und schwere (H) Immunglobulinketten und deren konstante, variable oder hypervariable Regionen sowie Fv- und Fd-Fragmente (Chamov et al., 1999, Antibody Fusion Proteins, Wiley-Liss Inc.). Die Antikörper können humanen oder nicht-humanen Ursprungs sein. Hierunter fallen beispielsweise die im Menschen bekannten Klassen: IgA, IgD, IgE, IgG and IgM, mit ihren verschiedenen Subklassen, beispielsweise IgA1, IgA2 and IgG1, IgG2, IgG3 and IgG4. Auch humanisierte und chimäre Antikörper kommen in Frage. Von besonderer therapeutischer Bedeutung und somit Gegenstand der vorliegenden Erfindung sind Pulverformulierungen die Antikörper gegen beispielsweise verschiedene Oberflächenantigene wie CD4, CD20 oder CD44, verschiedene Cytokine, beispielsweise IL2, IL4 oder IL5. Weitere Beispiele sind Antikörper gegen bestimmte Immunglobulin-Klassen (z.B. anti-IgE-Antikörper) oder gegen virale Proteine (zb. anti-RSV-, anti-CMV Antikörper, etc.).

Fab-Fragmente (Fragment antigen-binding = Fab) bestehen aus den variablen Regionen beider Ketten, die durch die angrenzenden konstanten Regionen zusammengehalten werden. Weitere Antikörperfragmente sind F(ab')₂-Fragmente, die durch proteolytischen Verdau mit Pepsin hergestellt werden können. Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Solche Antikörperfragmente werden auch als *single-chain* Fv-Fragment (scFv) bezeichnet. Beispiele von scFv-Antikörpern sind bekannt und beschrieben, siehe z.B. Huston et al., 1988, Proc. Natl. Acad. Sci. USA, 16, 5879ff.

In den vergangenen Jahren wurden verschiedene Strategien entwickelt um multimere scFv-Derivate herzustellen, wie z.B. Dia-, Tri- und Pentabodies. Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VH/VL-Ketten. Die Diabodies können zusätzlich durch eingeführte Disulfidbrücken stabilisiert werden. Beispiele von Diabodies finden sich in der Literatur, z.B. bei Perisic et al., 1994 (Structure, 2, 1217ff). Als "Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgG1, als Dimerisierungsregion enthält. Diese verbindet die scFv-Fragmente über eine Hinge-Region, ebenfalls von IgG, und eine Linker-Region. Beispiele solcher Minibodies sind bei Hu et al.,1996, Cancer Res., 56, 3055ff beschrieben. Mit "Triabody" bezeichnet der Fachmann ein trivalentes homotrimeres scFv-Derivat (Kortt et al., 1997, Protein Engineering, 10, 423ff). Die direkte Fusion von VH-VL ohne Verwendung einer Linkersequenz führt zur Ausbildung von Trimeren.

Bei den vom Fachmann als Mini-Antikörper bezeichneten Fragmenten, die eine bi-, trioder tetravalente Struktur haben, handelt es sich ebenfalls um Derivate von scFvFragmenten. Die Multimerisierung wird dabei über di-, tri- oder tetramere "coiled coil"-Strukturen erzielt (Pack, P. et al., 1993, Biotechnology, 11, 1271ff; Lovejoy, B. et al.,1993, Science, 259, 1288ff; Pack, P. et al., 1995, J. Mol. Biol., 246, 28ff).

Die sprühgetrockneten Pulver können ein Protein aus der Klasse der Antiköper, genauer Typ 1 Immunglobulin G., umfassen Es handelt sich dabei um einen humanisierten monoklonalen Antikörper, mit 95% humanen und 5% murinen Antikörpersequenzen. Der Antikörper hat ein Molekulargewicht von ca. 148 Kilodalton (kDa), bestehend aus zwei leichten und zwei schweren Ketten und insgesamt vier Disulfidbrücken.

In einem weiteren Aspekt enthalten die sprühgetrockneten Pulver als Wirkstoff ein Peptid oder Protein oder eine Kombination aus Peptid/Peptid, Peptid/Protein oder Protein/Protein. Die entsprechenden biologischen Makromoleküle können zwischen 0,01 bis 50% (w/w) der Trockenmasse des Pulver ausmachen. Der Anteil beträgt somit beispielsweise 0,01, 0,02, 0,03 ... 0,08, 0,09, 0,1, 0,2, 0,3 ... 0,8, 0,9 etc.; 1, 2, 3, ... 8 ,9, 10 etc.; 11, 12, 13, ... 18, 19, 20 etc.; 21, 22, 23, ... 28 ,29, 30 etc.; 31, 32, 33, ... 38, 39, 40 etc.; 41, 42, 43, ... 48, 49, 49,1, 49,2, 49,3, ... 49,8, 49,9 etc.; 49,91, 49,92, 49,93, ... 49,98, 49,99 (w/w).

Enthalten die erfindungsgemäßen Pulver sehr kleine Proteine/Peptide mit einem Molekulargewicht von < 10 kDa, vorzugsweise von < 5 kDa, wie zum Beispiel Wachstumsfaktoren, beispielsweise Cytokine, so beträgt der Anteil vorzugsweise zwischen 0,1 bis 10% (w/w), weiter bevorzugt zwischen 0,2 bis 5% (w/w) am Gesamtgewicht des Pulvers. Demnach sind Pulver bevorzugt deren Anteil an Cytokinen 0,2, 0,3, 0,4 ... 0,8, 0,9 etc.; 1, 2, 3, ... etc; 4,1, 4,2, 4,3, ... 4,8, 4,9 etc.; 4,91, 4,92, 4,93, ... 4,98, 4,99 (w/w) beträgt.

Handelt es sich bei dem pharmazeutischen Wirkstoff hingegen um einen oder mehrere Antikörper oder ein Derivat hiervon (bevorzugte Ausführungsform), so beträgt der Wirkstoffanteil am Feststoffgehalt des Pulvers zwischen 0,01 und 50% (w/w), vorzugsweise zwischen 0,1 und 50% (w/w), weiter bevorzugt zwischen 0,33 und 50% (w/w), beispielsweise 0,1, 0,2, 0,3, 0,33, ... 0,66, 0,7, 0,8, 0,9 etc.; 1, 2, 3, ... 8 ,9, 10 etc.; 11, 12, 13, ... 18, 19, 20 etc.; 21, 22, 23, ... 28 ,29, 30 etc.; 31, 32, 33, ... 38, 39, 40 etc.; 41, 42, 43, ... 48 ,49, etc; 49,1, 49,2, 49,3, ... 49,8, 49,9 etc.; 49,91, 49,92, 49,93, ... 49,98, 49,99 (w/w).

Nach einer besonderen Ausführungsform beträgt der Antikörperanteil am Feststoffgehalt des Pulvers zwischen 10 und 50% (w/w), weiter bevorzugt zwischen 10 und 30% (w/w), noch weiter bevorzugt zwischen 10 und 20% (w/w). Besonders 25 vorteilhaft und erfindungsgemäß sind Pulver, vorzugsweise sprühgetrocknete Pulver, mit einem Verhältnis von niedermolekularem Dextran zu Antikörper von 50/50, 51/49, 52/48, 53/47, 54/46, 55/45, 56/44, 57/43, 58/42, 59/41, 60/40, 61/39, 62/38, 63/37, 64/36, 65/35, 66/34, 67/33, 68/32, 69/31, 70/30, 71/29, 72/28, 73/27, 74/26, 75/25, 76/24, 77/23, 78/22, 79/21, 80/20, 81/19, 82/18, 83/17, 84/16, 85/15, 86/14, 87/13, 88/12, 89/11, oder 90/10 (w/w).

In diesem Zusammenhang gelten Pulver als erfindungsgemäß mit a) einem Anteil an niedermolekularem Dextran von zumindest 50% (w/w), vorzugsweise von 55 bis 98,99% (w/w), besonders bevorzugt von 60 bis 98,99% (w/w), b) einem Anteil von 1 bis 20% (w/w) eines Tri-Isoleucin und c) 0,01 bis maximal 49% (w/w) eines pharmazeutischen Wirkstoffs, vorzugsweise eines Peptids/Proteins.. Auch hier gilt, dass die Summe der Einzelfeststoffe 100% (w/w) nicht übersteigen kann. Erfindungsgemäß sind ferner Pulver mit folgender Zusammensetzung: 89% (w/w) niedermolekulares Dextran/ 1% Tri-Isoleucin/ 10% (w/w) pharmazeutischer Wirkstoff (89/1/10); (88/2/10); (87/3/10); (86/4/10); (85/5/10); (84/6/10); (83/7/10); (82/8/10); (81/9/10); (80/10/10); (79/11/10); (78/12/10); (77/13/10); (76/14/10); (75/15/10); (74/16/10);, (73/17/10); (72/18/10) oder (71/19/10), wobei der Anteil am pharmazeutischen Wirkstoff auch von 10 auf 0,01 % (w/w), beispielsweise auf 9,99, ... 9,9, 9,8, 9,7 ... 9,3, 9,2, 9,1 ... 9, 8 7, 6, 5, 4, 3, 2, 1, ...0,9, 08, 0,7, ... 0,66, ... 0,6, 0,5, 0,4, 0,3, 0,2, 0,1, 0,09, 0,08, 0,07, 0,06, 0,05, 0,04, 0,03 0,02, 0,01% (w/w) reduziert werden kann und sich dementsprechend der Anteil an niedermolekularen Dextran auf beispielsweise 80,01, ... 80,1, 80,2, 80,3 ... 80,8,
80,9, 81, 82, 83, 84, 85, 86, 87, 88, 89, ... 89,1, 89,2, 89,3, ... 89,33, ... 89,4, 89,5, 89,6 , 89,7, 89,8, 89,9, ... 89,91, 89,92, 89,93, ... 89,97, 89,98, 89,99% (w/w) erhöhen kann, so dass die Summe der Gewichtsanteile in Bezug auf die Trockenmasse des Pulvers 100% (w/w) ergibt. Erfindungsgemäß sind demnach auch Pulver mit folgender Zusammensetzung: 80% (w/w) niedermolekulares Dextran/ 19% (w/w) Tri-Isoleucin / 1% (w/w) pharmazeutischer Wirkstoff (80/19/1); (80/18/2); (80/17/3); (80/16/4); (80/15/5); (80/14/6); (80/13/7); (80/12/8); (80/11/9) ; (80/10/10); (70/19/11); (70/18/12); (70/17/13); (70/16/14); (70/15/15); (70/14/16); (70/13/17); (70/12/18); (70/11/19); (70/10/20); (60/19/21); (60/18/22); (60/17/23); (60/16/24); (60/15/25); (60/14/26); (60/13/27); (60/12/28); (60/11/29); (60/10/30), wobei der Anteil an Tri-Isoleucin auch von 10 auf 1 % (w/w), beispielsweise auf 9,99, ... 9,9, 9,8, 9,7 ... 9,3, 9,2, 9,1 ... 9, 8 7, 6, 5, 4, 3, 2, 1,9, 1,8, 1,7, ... 1,66, ... 1,6, 1,5, 1,4, 1,3, 1,2, 1,1, 1% (w/w) reduziert werden kann und dementsprechend der Anteil an pharmazeutischen Wirkstoff, vorzugsweise an Peptid/Protein auf beispielsweise 30,1, 30,2, 30,3 ... 30,8, 30,9, 31, 32, 33, 34, 35, 36, 37, 38, 38,1,.38,2, 38,3, ... 38,33, ..., 38,4, 38,5, 38,6 , 38,7, 38,8, .38,9, ... 39 (w/w) erhöht werden kann, so dass die Summe der Gewichtsanteile in Bezug auf die Trockenmasse des Pulvers 100% (w/w) ergibt. Bei Reduktion des Anteils an Tri-Isoleucin von 10 auf 1 (w/w), wie hier dargestellt, kann auch der an niedermolekularem Dextran im Pulver erhöht werden. Bei beispielsweise konstantem Wirkstoffanteil von 10% (w/w) lassen sich so Pulver mit einem Dextrananteil von 80,1, 80,2, 80,3 ... 80,8, 80,9, 81, 82, 83, 84, 85, 86, 87, 88, 88,1, 88,2, 88,3, ... 88,33, ..., 88,4, 88,5, 88,6 , 88,7, 88,8, 88,9 bzw. 89 (w/w) herstellen.

Nach einer weiteren erfindungsgemäßen Ausführungsform können die Pulver zusätzlich oberflächenaktiven Substanzen wie Tween 20, 40, 60, 80, Brij 35, Pluronic F 88 und Pluronic F 127 enthalten. Diese werden vorzugsweise in einer Konzentration von 0,01-0,1% (w/w) eingesetzt. Besonders bevorzugt ist ein sprühgetrocknetes Pulver dass als Hilfsstoff niedermolekulares Dextran und zusätzlich Tween 20, vorzugsweise in einer Konzentration von 0,01-0,1% (w/w), als oberflächenaktive Substanz enthält.

Nach einer weiteren Ausführungsform betrifft die vorliegende Erfindung auch pharmazeutische Zusammensetzung enthaltend eines der oben beschriebenen sprühtgetrockneten Pulver.

### Herstellung der Erfindungsgemäßen Pulver:

Die vorliegende Erfindung stellt auch Verfahren zur Herstellung eines der oben näher beschriebenen sprühgetrockneten Pulvers zur Verfügung. Das Verfahren ist dadurch gekennzeichnet, dass eine zu versprühende Lösung / Suspension enthaltend einen pharmazeutischen Wirkstoff, ein Tri-Isoleucin und niedermolekulares Dextran unterhalb von einer Temperatur von 200/120°C (Einström-/Auslasstemperatur) vorzugsweise bei 150-185/70-95°C versprüht wird. Der erfindungsgemäße Prozess ist im Abschnitt "BEISPIELE" anhand einiger Beispiele näher beschrieben.

Grundsätzlich lassen sich die erfindungsgemäßen Pulver herstellen, in dem der pharmazeutische Wirkstoff ein Tri-Isoleucin, vorzugsweise ein biologisches Makromolekül, in Form eines Peptids oder Proteins, in einer wässrigen Lösung, abhängig von den Löslichkeitsbedingungen des jeweiligen Wirkstoffs, gelöst wird. Zumeist werden gepufferte Lösungen mit einem pH von 3-11, vorzugsweise von 3,5-9 verwendet. Bei der Herstellung von inhalierbaren Pulvern ist eine wässrige Lösung mit einem pH von 4-7,8 besonders vorteilhaft. Um eine hinreichende Löslichkeit zu gewähren, sollte der pH-Wert der Lösung unterhalb des pl-Werts des Peptids/Proteins liegen. Die wässrige Lösung kann optional zusätzliche wasserlösliche organische Lösungsmittel enthalten, wie z.B. Aceton, Alkohole oder der gleichen. Besonders geeignet sind niedere Alkohole wie z.B. Methanol, Ethanol, Propanol, (n- oder iso-Propanol) oder der gleichen. Solche gemischten Lösungsmittelsysteme enthalten normalerweise zwischen 10-20% (v/v) eines wasserlöslichen organischen Lösungsmittels. Der Feststoffanteil in der zu versprühenden Lösung beträgt üblicherweise zwischen 0,01-20% (w/w), vorzugsweise zwischen 0,05-10% (w/w), besonders bevorzugt zwischen 0.1-5% (w/w). In Rahmen der vorliegenden Erfindung wurden sprühgetrocknete Pulver ausgehend von einer wässrigen Lösung mit einem Feststoffanteil von 10% (w/w) bzw. 3,33% (w/w) hergestellt.

Üblicherweise wird der Hilfsstoff oder eine Mischung aus geeigneten Hilfsstoffen, wie oben beispielhaft aufgeführt, in einem zweiten Behältnis in Reinstwasser oder einer geeigneten Pufferlösung mit einem pH-Wert von 3 bis 11, bevorzugt von 3,5 bis 9 und besonders bevorzugt von 4,0 bis 7,8 gelöst und in einem zweiten Schritt mit der Wirkstofflösung vermischt. Anschließend wird die Lösung / Suspension mit Reinstwasser oder einer geeigneten Pufferlösung mit einem pH-Wert von 3 bis 11, bevorzugt von 3,5 bis 9 und besonders bevorzugt von 4,0 bis 7,8 auf den gewünschten Feststoffgehalt eingestellt.

Folglich betrifft die vorliegende Erfindung ein Verfahren zur Herstellung eines sprühgetrockneten Pulvers dadurch gekennzeichnet, dass
a) ein pharmazeutischer Wirkstoff in einer wässrigen Lösung / Suspension gelöst wird;
b) niedermolekulares Dextran in einer wässrigen Lösung / Suspension gelöst wird;
c) die Lösung oder Suspension neben dem niedermolekularen Dextran und dem pharmazeutischen Wirkstoff zumindest ein Tri-Isoleucin als Hilfsstoff enthält und sofern Wirkstoff, Tri-Isoleucin und niedermolekulares Dextran in verschiedenen Lösungen / Suspension gelöst wird, diese gemischt werden;
d) die Lösung / Suspension enthaltend niedermolekulares Dextran und den pharmazeutischen Wirkstoff unterhalb von einer Temperatur von 200/120°C vorzugsweise von 175/95°C versprüht wird.

Die erfindungsgemäße zu versprühende Lösung enthält neben niedermolekularem Dextran Tri-Isoleucin. Als vorteilhaft gelten zu versprühende Lösungen oder Suspension deren Feststoffanteil a) zumindest 50% (w/w), vorzugsweise 60 bis 98,99% (w/w) niedermolekulares Dextran, b) 1 bis 19% (w/w) Tri-Isoleucin, und c) zumindest 0,01% (w/w) eines pharmazeutischen Wirkstoffs, vorzugsweise eines Peptids/Proteins wie zum Beispiel eines Antikörpers, enthält, wobei die Summe aus den Feststoffanteilen maximal 100 % (w/w) beträgt. Der Anteil de pharmazeutischen Wirkstoffs beträgt hierbei vorzugsweise 0,01 bis maximal 39% (w/w). Ein Fachmann ist hierbei in der Lage entsprechende Pulver herzustellen und die Gewichtsanteile aufeinander abzustimmen, so dass die Summe aus den Feststoffanteilen 100% (w/w) nicht übersteigt. Soll der Anteil (bezogen auf den Gesamtfeststoffgehalt) an pharmazeutischen Wirkstoff beispielsweise 30% (w/w) betragen und der Anteil an niedermolekularem Dextran 60% (w/w) so weiß der Fachmann, das er der zu versprühenden Lösung oder Suspension maximal 10% (w/w) an Tri-Isoleucin zugeben kann.

Wie bereits erwähnt ist es vorteilhaft zu versprühende Lösungen mit einem pH-Wert zwischen 3 und 11, vorzugsweise 3,5 und 9, besonders bevorzugt zwischen 4,0 und 7,8 herzustellen und zu versprühen. Geeignete Puffersysteme sind dem Fachmann bekannt. Üblicherweise erweist sich die Verwendung von anorganischen oder organischen Salzen als Puffersystem als besonders vorteilhaft.

Typischerweise wird der optimale Hilfsstoff- und Proteingehaltgehalt für jedes Protein oder Peptid experimentell bestimmt. Bevorzugte Formulierungen der Erfindung können noch mindestens einen weiteren Hilfsstoff enthalten, um Pulvereigenschaften wie Dispergierbarkeit und Fließfähigkeit unter Beibehaltung einer überlegenen Aggregathemmung zu verbessern.

Die Versprühung erfolgt in konventionellen Sprühtrocknern, beispielsweise in Geräten der Fa. Niro A/S (Soeborg, DK), Büchi Labortechnik GmbH (Flawil, CH) oder der gleichen. Die optimalen Bedingungen für die Sprühtrocknung hängen jeweils von der entsprechenden Formulierung ab und sind experimentell zu bestimmen. Als Gas wird typischerweise Luft verwendet, geeignet sind aber auch inerte Gase wie Stickstoff oder Argon. Darüber hinaus bestimmt sich die Sprühtrocknungstemperatur, gemeint sind Einström- *(inlet-)* und Ausströmtemperatur (*outlet*-temperature), nach der Temperatursensitivität des verwendeten Wirkstoffs, jeweils in Abhängigkeit der verwendeten Stabilisatoren. Üblich ist eine *inlet*-Temperatur von 50-200°C während die *outlet-*Temperatur zumeist 30-150°C beträgt. Im Rahmen der vorliegenden Erfindung wurde mit einer *inlet*-Temperatur von ungefähr 170-185°C und einer *outlet*-Temperatur von 80-100°C gearbeitet. Möglich sind allerdings auch etwas höhere Temperaturen, beispielsweise eine *inlet*-Temperatur von bis zu 200°C, vorzugsweise 90-185°C und eine *outlet*-Temperatur von bis zu 120°C, vorzugsweise 90-105°C, je nach Stabilisatoranteil. Die Versprühung erfolgt in der Regel bei einem Druck von ungefähr 20-150 psi, vorzugsweise bei ungefähr 30- oder 40-100 psi, beispielsweise bei ungefähr 30, 40, 50, 60, 70, 80, 90 oder 100 psi.

In Bezug auf den Büchi-Sprüher beträgt die "Liquid-Feed-Rate" normalerweise zwischen 0,1 und 100 ml/min, vorzugsweise zwischen 0,1 und 30 ml/min, beispielsweise ca. 3 ml/min. In diesem Zusammenhang hat sich eine Aspirator-Flow-Rate von 20-40 m³/h, vorzugsweise von 30-40 m³/h wie zum Beispiel 35 m³/h und eine Zerstäubungsflussrate von 0,3-2,5 m³/h vorzugsweise von ca. 0,67 m³/h als besonders geeignet erwiesen.

Die sprühgetrockneten Wirkstoffformulierungen, vorzugsweise die Protein-Pulverformulierungen können optional einer zweiten schonenden Trocknung (Nachtrocknung) unterzogen werden. Ziel ist es einen einheitlichen Restwassergehalt der Formulierungen von kleiner 2% (w/w) zu erhalten, und damit sowohl die Wirkstoffstabilität als auch Pulvereigenschaften wie Glasübergangstemperatur, Fließfähigkeit und Dispergierbarkeit zu verbessern. Die Bedingungen des Nachtrocknungsprozesses müssen so gewählt sein, dass sich die Aggregatbildung des Wirkstoffs nicht signifikant erhöht. Dies trifft insbesondere auf die Verwendung von biologischen Makromolekülen, wie beispielsweise auf die Verwendung von Peptiden/Proteinen zu. Die sprühgetrockneten Wirkstoff-Pulverformulierungen werden bevorzugt unter trockenen Bedingungen (bei geringer relativer Luftfeuchtigkeit) hergestellt, weiterverarbeitet und gelagert. Der Prozess der Nachtrocknung ermöglicht es die Pulver bei relativ hohen Luftfeuchtigkeiten herzustellen und abzufüllen. Überraschenderweise stabilisieren die Hilfsstoffe, welche Gegenstand der Erfindung sind, in den bevorzugten Formulierungen die Proteine auch bei nicht optimalen Prozess- und Lagerbedingungen überlegen.

### Eigenschaften der sprühgetrockneten trockenen Pulverformulierungen

Die im Rahmen dieser Erfindung hergestellten trockenen Protein-Pulverformulierungen haben einen Restwassergehalt von unter 15% (w/w), gewöhnlich von unter 10% (w/w), und bevorzugt von unter 6% (w/w). Weiter bevorzugt haben die sprühgetrockneten Protein-Pulverformulierungen einen Restwassergehalt von unter 3% (w/w), besonders bevorzugt von unter 2% (w/w) und am meisten bevorzugt von zwischen 0,2 und 2,0% (w/w). Formulierungen mit einer geringen Restfeuchte zeigen generell eine verbesserte Stabilität während des Abpackens und der Lagerung. Darüber hinaus sind die die trockenen Protein-Pulverformulierungen der Erfindung vornehmlich hygroskopisch, d.h. sie neigen dazu Feuchtigkeit aus Ihrer Umgebung zu absorbieren. Um dies zu vermeiden werden solche Pulver für gewöhnlich unter Ausschluss von Luftfeuchtigkeit in Behältern wie Blisterpackungen gelagert.

Die stabilisierenden Effekte der hier beschriebenen Hilfsstoffe sind imstande das Protein vor den extremen Belastungen während der Sprühtrocknung sowie der Lagerung zu schützen. In Abwesenheit von Hilfsstoffen sprühgetrocknete pure Proteinformulierungen bilden in großem Ausmaß Aggregate. Prozessbedingte Faktoren wie Hitze, Scherstress und Denaturierung an den Luft-Wasser-Grenzflächen verursachen Aggregation (bis zu ca. 3,7% Aggregate) während der Sprühtrocknung und angeschlossener Nachtrocknung (bis zu ca. 4,0% Aggregate). Im Verlauf der Lagerung kommt es durch Abwesenheit der stabilisierenden Hydrathülle der Proteine zu massiver Aggregatbildung (von ca. 11,8 bis ca. 18,9 % Aggregate).

Die bevorzugten sprühgetrockneten Formulierungen der Erfindung sind im Gegensatz zu den puren Proteinformulierungen imstande die Bildung von Aggregaten sowohl nach dem Sprühtrocknen zu verringern als auch bei unterschiedlichen Lagerbedingungen auf einem sehr geringen Niveau zu halten. Durch die Sprühtrocknung bilden sich in den bevorzugten Formulierungen lediglich ca. 1,1 bis ca. 1,4% Aggregate, im Gegensatz zu ca. 4,0% Aggregate bei puren Proteinformulierungen. Bevorzugte Formulierungen, die einer zweiten schonenden Trocknung unterzogen werden, zeigen keine Tendenz zu einer verstärkten Aggregatbildung. Bei einer besonders herausfordernden Lagerungsbedingung (40°C, 75% relative Feuchte) zeichnet sich eine deutliche Überlegenheit der bevorzugten Formulierungen (Aggregate von ∼5,1 bis -10,1%) gegenüber puren Proteinformulierungen ab (ca. 18,9% Aggregate) sowie einer analogen Referenzformulierung mit Trehalose als Hilfsstoff ab.

Formulierungen, welche bereits bei relativ kurzer Lagerung unter besonders destabilisierenden Bedingungen (1 Woche bei 40°C, 75% relative Feuchte) einen signifikant stabilisierenden Effekt auf die inkorporierten Proteine haben, stabilisieren Proteine auch auf lange Zeit unter weitaus milderen Standardlagerbedingungen (z.B. 1 Jahr trocken, ca. 25°C).

Durch Variation der Sprühtrocknungsbedingungen lassen sich Pulver herstellen, die vorzugsweise über eine mittlere Teilchengröße (MMD) von weniger als 20 *µ*m, vorzugsweise von weniger als 10 *µ*m verfügen. Nach einer besonders bevorzugten Ausführungsform verfügen diese erfindungsgemäßen Partikel über einen mittlere Teilchengröße von weniger als 7,5 *µ*m; vorzugsweise von weniger als 5 *µ*m. Besonders bevorzugt sind Partikel mit einem mittlere Teilchengröße von weniger als 4 *µ*m und weiter bevorzugt von weniger als 3,5 *µ*m. Im allgemeinen lassen sich auch Partikel mit einen mittleren Teilchendurchmesser von 0,1-5 *µ*m, vorzugsweise von 0,2-4 *µ*m herstellen. In einer weiteren Ausführungsform werden den entsprechenden Pulvern nicht-respirierbare Teilchen, z.B. Laktose, mit einer Teilchengröße von zumindest 40 *µ*m, vorzugsweise zwischen 40 und 200 *µ*m, zugemischt.

Neben der mittleren Teilchengröße (MMD) hängt die Inhalierbarkeit im wesentlichen von dem mittleren aerodynamischen Teilchendurchmesser (MMAD) ab. Die erfindungsgemäßen Partikel verfügen vorzugsweise über einen MMAD von kleiner 10 *µ*m und weiter bevorzugt von weniger als 7,5 *µ*m. Besonders vorteilhaft sind Pulver bestehend aus Partikeln mit einem MMAD von kleiner 5,5 *µ*m, vorzugsweise von kleiner 5 *µ*m, noch weiter bevorzugt von kleiner 4,5 *µ*m. Die in den Beispielen beschriebenen Pulver lassen sich mit entsprechenden Partikelgrößen durch die Kombination aus optimaler Sprühtrocknungsbedingung und der erfindungsgemäßen Wahl und Konzentration der Hilfsstoffe herstellen. Insbesondere die Beimischung von Aminosäuren und/oder Tri-Peptiden führt zu einer verbesserten Partikelperfomance mit einem erhöhten Anteil an inhalierbaren Partikeln mit einem MMAD von klein 7,5, vorzugsweise von kleiner 5,5. Durch Zugaben von Isoleucin oder Tri-Isoleucin konnten inhalierbare Pulver mit einer FPF von größer 30%, vorzugsweise von größer 40, weiter bevorzugt von größer 50 und noch weiter bevorzugt von größer 55% hergestellt werden (siehe BEISPIELE).

Die erfindungsgemäßen Pulver zeichnen sich darüber hinaus durch eine Glasübergangstemperatur von zumindest 45°C, vorzugsweise von zumindest 50°C, weiter bevorzugt von zumindest 55°C, noch weiter bevorzugt von zumindest 60°C aus. Besonders bevorzugte Pulver weisen eine Glasübergangstemperatur von zumindest 65°C, auf. Im allgemeinen beträgt die Glasübergangstemperatur der erfindungsgemäßen detranhaltigen Pulver 60 bis 65°C. Demzufolge betrifft die vorliegende Erfindung auch Pulver, vorzugsweise sprühgetrocknete Pulver, enthaltend einen pharmazeutischen Wirkstoff und niedermolekulares Dextran, wobei die Glasübergangstemperatur 45°C und mehr, vorzugsweise zwischen 45 und 70 C beträgt. Nach einer weiter bevorzugten Ausführungsform beträgt die Glasübergangstemperatur 55°C und mehr, vorzugsweise zwischen 55 und 70°C.

### Verwendung des sprühgetrockneten Pulvers

Die erfindungsgemäßen Pulver eignen sich zur Herstellung eines Arzneimittels, vorzugsweise zur Herstellung eines inhalativen Arzneimittels.

### Verabreichung der erfindungsgemäßen Pulver

Grundsätzlich lassen sich die erfindungsgemäßen Pulverzubereitungen direkt als trockenes Pulver über sog. Trockenpulver-Inhalatoren, oder aber nach Rekonstitution in Form von Aerosolen über sog. Vernebler applizieren. Die erfindungsgemäßen Inhalationspulver können dabei mittels aus dem Stand der Technik bekannten Inhalatoren appliziert werden.

Erfindungsgemäße Inhalationspulver können beispielsweise mittels Inhalatoren appliziert werden, die eine einzelne Dosis aus einem Vorrat mittels einer Messkammer, wie er in der US 4,570,630A beschrieben wird, oder über andere apparative Vorrichtungen, wie sie in der DE 36 25 685 A beschrieben werden, appliziert werden. Vorzugsweise werden die erfindungsgemäßen Inhalationspulver in Kapseln abgefüllt (zu sogenannten Inhaletten), die in Inhalatoren, wie beispielsweise in der WO 94/28958 beschrieben, zur Anwendung gelangen.

Weitere Beispiele für geeignete Inhalatoren finden sich u.a. in US 5,458,135; US 5,785,049 oder WO 01/00263. Weitere geeignete Inhalatoren sind aus der WO 97/41031; US 3,906,950 und US 4,013,075 bekannt. Weitere Dispersionsinhalatoren für trockene Pulverzubereitungen sind in EP 129 985; EP 472 598; EP 467 172 und US 5,522,385 beschrieben.

Die erfindungsgemäßen Inhalationspulver können beispielsweise mittels des unter dem Namen Turbuhaler® (AstraZeneca LP) bekannten Inhalators beziehungsweise mit Inhalatoren wie sie beispielsweise in der EP 237 507 A offenbart werden, appliziert werden. Andere geeignete Inhalatoren sind der Rotahaler® oder der Discus® (beide von GlaxoSmithKline Corp.), der Spiros™ Inhaler (Dura Pharmaceuticals) und der Spinhaler® (Fiscon).

Ein zur Anwendung der erfindungsgemäßen Arzneimittelkombination in Inhaletten besonders bevorzugter Inhalator ist der Figur 12 zu entnehmen. Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlassöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12, sowie Luftdurchlasslöcher 13 zur Einstellung des Strömungswiderstandes.

Sollen die erfindungsgemäßen Inhalationspulver im Sinne der vorstehend genannten bevorzugten Anwendung in Kapseln (Inhaletten) abgefüllt werden, bieten sich Füllmengen von 1 bis 30mg pro Kapsel an.

Die erfindungsgemäßen Pulver können ferner als treibgashaltige Inhaltionsaerosole appliziert werden. Hierzu werden die erfindungsgemäßen Pulver in einer wässrigen Lösung rekonstituiert. Geeignete Lösungen sind im Stand der Technik bekannt. Vorteilhaft ist beispielsweise die Rekonstitution in physiologischen Lösungen mit einem pH von 3-11, vorzugsweise von 4-9. Besonders vorteilhaft ist die Rekonstitution in einer wässrigen Lösung mit einem pH von 5,5-7,8. Die Lösung zur Rekonstitution der erfindungsgemäßen Pulver kann ferner weitere Hilfsstoffe in Form von Stabilisatoren, Emulgatoren oberflächenaktiven Substanzen, wasserlöslichen organischen Lösungsmitteln enthalten. Entsprechende Substanzen sind dem Fachmann bekannt, und beispielhaft in (Bauer, Lehrbuch der Pharmazeutischen Technologie, Wissenschaftl. Verlagsgesellschaft mbH, Stuttgart, 178-184; Adler, 1998, Journal of Pharmaceutical Sciences, 88(2), 199-208) beschrieben.

Die zur Herstellung der Inhalationsaerosole einsetzbaren Treibgase sind ebenfalls aus dem Stand der Technik bekannt. Geeignete Treibgase sind ausgewählt aus der Gruppe bestehend aus Kohlenwasserstoffen wie n-Propän, n-Butan oder Isobutan und Halogenkohlenwässerstoffen wie bevorzugt chlorierten und fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG11, TG12, TG134a (1,1,1,2- Tetrafluorethan), TG227 (1,1,1,2,3,3,3-Heptafluorpropan) und Mischungen derselben, wobei die Treibgase TG134a, TG227 und Gemische derselben bevorzugt sind.

Die treibgashaltigen Inhalationsaerosole können bis zu 5% (w/w) an Wirkstoff enthalten. Aerosole enthalten beispielsweise 0,002-5%(w/w), 0,01-3% (w/w), 0,015-22/0 (w/w), 0,1-2% (w/w), 0,5-2% (w/w) oder 0,5-1% (w/w) an dem pharmazeutischen Wirkstoff. Inhaltationsaerosole mit einer entsprechenden Wirkstoffkonzentration lassen sich durch gezielte Rekonstitution der erfindungsgemäßen Pulver in einer entsprechenden Menge an Lösungsmittel einstellen.

Die vorstehend genannten treibgashaltigen Inhalationaerosole können mittels im Stand der Technik bekannten Inhalatoren (MDIs = metered dose inhalers) appliziert werden. Beispielhaft sei hier auf den Ventolin® (Ventolin Pharmacy) oder die in US 5,32,094 oder US 5,672,581 beschriebenen Inhalatoren verwiesen. Dementsprechend betrifft ein weiterer Aspekt Arzneimittel in Form von wie vorstehend beschriebenen treibgashaltigen Aerosolen in Verbindung mit einem oder mehreren zur Verabreichung dieser Aerosole geeigneten Inhalatoren.

Kartuschen, die ausgestattet mit einem geeigneten Ventil in einem geeigneten Inhalator zur Anwendung gelangen, können _eine der vorstehend genannten treibgashaltigen Inhalationsaerosole enthalten. Geeignete Kartuschen und Verfahren zur Abfüllung dieser Kartuschen mit den treibgashaltigen Inhaltionsaerosolen sind aus dem Stand der Technik bekannt.

Die erfindungsgemäßen Pulver können ferner in treibgasfreien Inhalationslösungen oder Suspensionen rekonstituiert werden. Entsprechende treibgasfreie Inhalationslösungen enthalten beispielsweise wässrige oder alkoholische, bevorzugt ethanolische, gegebenenfalls ethanolische im Gemisch mit wässrigen Lösungsmitteln. Im Falle wässrig/ethanolischer Lösungsmittelgemische ist der relative Anteil an Ethanol gegenüber Wasser nicht begrenzt, bevorzugt liegt die maximale Grenze jedoch bei bis 70% (v/v), insbesondere bei bis zu 60% (v/v) Ethanol. Die restlichen Volumenprozente werden von Wasser aufgefüllt. Den erfindungsgemäßen treibgasfreien Inhalationslösungen können Co-Solventien und/oder weitere Hilfsstoffe, wie oben beschrieben, zugesetzt werden. Beispielsweise lassen sich Co-Solventien verwenden, die Hydroxylgruppen oder andere polare Gruppen enthalten, wie z.B. Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen neben den oben aufgeführten z.B. oberflächenaktive Stoffe, wie z.B. Sojalecithin, Ölsäure, Sorbitanester, wie Polysorbate, Polyvinylpyrrolidon sonstige Stabilisatoren,
Komplexbildner, Antioxidantien und/oder Konservierungsstoffe, die die Verwendungsdauer der fertigen Arzneimittelformulierung gewährleisten oder verlängern, Geschmacksstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100m1, besondersbevorzugt zwischen 5 und 20 mg/100m1 enthalten. Demnach umfasst ein weiterer Aspekt treibgasefreie Inhaltationsaerosole, die durch Rekonstituion der erfindungsgemäßen Pulver hergestellt werden.

Zur Applikation der treibgasfreien Inhaltionslösungen sind besonders solche Inhalatoren geeignet, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Beschreibung sind solche Vernebler bevorzugt, bei denen bereits eine Menge von weniger als 100 µL, weniger als 50 µL, zwischen 10 und 30 µL Wirkstofflösung mit einem Hub zu einem Aerosol mit einer durchschnittlichen Teilchengröße von weniger als 20 µm, weniger als 10 µm, so vernebelt werden können, so dass der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere in den Figuren 6a und 6b) -ausführlich beschrieben. Auf die entsprechenden Figuren 6a und 6b der WO 97/12687 einschließlich der dazugehörigen Beschreibungsteile wird im Rahmen der vorliegenden Erfindung ausdrücklich Bezug genommen. Die dort beschriebenen Vernebler (Devices) sind auch unter der Bezeichnung Respimat® (Boehringer Ingelheim Pharma) bekannt. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so dass inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird im Rahmen der vorliegenden Erfindung auf die Figuren 1-4 - insbesondere auf Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO 94/07607 offenbart; auf diese Schrift wird hiermit inhaltlich Bezug genommen, insbesondere auf die dort offenbarte Figur 1 und deren Beschreibung. Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlassseite mit der Düsenauslassseite verbinden. Auf der Düsenauslassseite ist mindestens eine runde oder nicht-runde Öffnung von 2-10 *µ*m Tiefe und 5-15 *µ*m Breite, wobei die Tiefe bevorzugt bei 4, 5 bis 6,5 Mikrometern und die Länge bei 7-9 Mikrometern beträgt. Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslassseite können die Strahlrichtungen mit einem Winkel von 20-160 Grad gegeneinander geneigt sein, bevorzugt wird ein Winkel von 60-150 Grad, insbesondere bevorzugt 80-100°. Die Düsenöffnungen sind bevorzugt in einer Entfernung von 10-200 *µ*m angeordnet, stärker bevorzugt in einer Entfernung von 10-100 *µ*m, besonders bevorzugt 30-70 *µ*m. Am stärksten bevorzugt sind 50 *µ*m.

Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 *µ*m, bevorzugt bei 3-10 *µ*m.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 6 a/b der WO 97/12687 einschließlich der dazugehörigen Beschreibung, auf die an dieser Stelle nochmals inhaltlich Bezug genommen wird, ist ein entsprechender Vernebler (Respimat®) beschrieben. Dieser eignet sich insbesondere zur Applikation der erfindungsgemäßen treibgasfreien Inhalationsaerosole.

Figur 6a zeigt der WO97/12687 zeigt einen Längsschnitt durch den Zerstäuber bei gespannter Feder, Figur 6b der WO97/12687 zeigt einen Längsschnitt durch den Zerstäuber bei entspannter Feder: Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen. Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht. In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inhalatoren, beispielsweise Jet-Stream-Inhalatoren oder anderen stationären Verneblern vernebelt werden.

Dementsprechend betrifft ein weiterer Aspekt Arzneimittel in Form von wie vorstehend beschriebenen treibgasfreien Inhaltionslösungen oder Suspensionen in Verbindung mit einer zur Verabreichung dieser Formulierungen geeigneten Vorrichtung, bevorzugt in Verbindung mit dem Respimat®. Bevorzugt kommen treibgasfreie Inhaltionslösungen oder Suspensionen zum Einsatz, enthaltend eines der erfindungsgemäßen Pulver, in Verbindung mit der unter der Bezeichnung Respimat® bekannten Vorrichtung. Ferner ist vorstehend genannte Vorrichtungen zur Inhalation, bevorzugt den Respimat®, dadurch gekennzeichnet, dass sie vorstehend beschriebene treibgasfreie Inhalationslösungen oder Suspensionen enthalten.

In einem weiteren Aspekt enthalten Inhalationslösungen, eines der hier beschriebenen erfinddungsgemäßen Pulver, in einer einzigen Darreichungsform.

Die treibgasfreien Inhalationslösungen oder Suspensionen können neben den vorstehend, zur Applikation im Respimat® vorgesehenen Lösungen und Suspensionen auch als Konzentrate oder sterile gebrauchsfertige Inhalationslösungen bzw. -suspensionen vorliegen. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt Arzneimittel in Form von wie vorstehend beschriebenen treibgasfreien Inhaltionslösungen oder Suspensionen, die als Konzentrate oder sterile gebrauchsfertige Formulierungen vorliegen, in Verbindung mit einer zur Verabreichung dieser Lösungen geeigneten Vorrichtung, dadurch gekennzeichnet, dass es sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Weitere geeignete Vernebler für die inhalative Applikation von rekonstituierten Aerosolen sind der AERx™ (Aradigm), der Ultravent® (Mallinkrodt) und der AconII® (Maquest Medical Products).

### Beispiele

### Material und Methoden

### Materialien

Als IgG1 wurde ein humanisierter monoklonaler Antikörper mit einem Molekulargewicht von ca. 148 kDa verwendet. Der Antikörper ist von einem murinen Antikörper abgeleitet, in dem die komplementärbestimmenden Regionen des murinen Antikörpers auf ein humanes Immunglobulingerüst übertragen wurden. Es entstand ein chimärer Antikörper mit 95% humanen und 5% murinen Anteil. Der Antikörper wird aus murinen Myelomazelllinien exprimiert. Die Zellen werden mit Hilfe von Tangential Fluss Microflitration entfernt und die zellfreie Lösung durch verschiedene Chromatographiemethoden aufgereinigt. Weitere Schritte umfassen eine Nucleasebehandlung, Behandlung bei niedrigem pH-Wert und eine Nanofiltration. Die Antikörper enthaltende Bulklösung enthält Histidin 25 mM und Glycin 1,6 mM als Puffer und wurde für die Herstellung der Lösung zur Sprühtrocknung mittels Diafiltration auf ca. 100mg/ml aufkonzentriert. Der Bulk für die Herstellung der zu versprühenden Lösung besaß 0,8% Aggregate. Das Fertigarzneimittel ist bei 2-8°C mindestens 2 Jahre haltbar. Niedermolekulares Dextran1 oder auch Dextran₁₀₀₀ mit einem mittleren Molekulargewicht von ca. 1000 Da wurde von Amersham Biosciences AB, Uppsalla, Sweden bezogen. Trehalose stammt von der Georg Breuer GmbH, Deutschland. L-Isoleucin wurde von Sigma-Aldrich Chemie GmbH, Deutschland, bezogen. Tri-Isoleucin wurde von Iris Biotech GmbH, Deutschland bezogen. Hühnereiweiss-Lysozym (Lysozym), 135500 U/mg, wurde von SERVA Electrophoresis GmbH, Deutschland bezogen. Synthetisches Lachscalcitonin (Calcitonin) wurde von Biotrend Chemikalien GmbH, Deutschland bezogen.

### Sprühtrocknung mit Büchi B-290

Die Sprühtrocknung wurde mit Hilfe eines Büchi Mini Spray Dryer B-290 der Firma Büchi Labortechnik GmbH durchgeführt. Die Sprühtrocknung der Formulierungen wurden prinzipiell gemäß der Beschreibung in "Spray Drying Handbook", 5th Edition., K. Masters, John Wiley and Sons , Inc., NY, NY (1991) ausgeführt:
Der Sprühtrockner ist aus einem Heizsystem, einem Filter, einem Aspirator, einem Trocknungsturm, einem Zyklon, Temperatursensoren zur Messung der Einlass- und der Auslasstemperatur und einem Auffanggefäß aufgebaut. Mit Hilfe einer peristaltischen Pumpe wird die zu versprühende Lösung in eine Zwei-Stoffdüse gepumpt. Dort findet mittels Druckluft die Zerstäubung der Lösung in kleine Tropfen statt. Die Trocknung erfolgt im Sprühturm durch erwärmte Luft, die im Gleichstromverfahren mit Hilfe des Aspirators durch den Sprühturm gesaugt wird. Das Produkt wird im Auffanggefäß nach Passieren des Zyklons aufgefangen.

Es wurden zwei verschieden Zyklone verwendet:

| | |
|---|---|
| • Zyklon I: Büchi Cyclone | (Produktnummer 4189) |
| • Zyklon II: Büchi High-performance Cyclone | (Produktnummer 46369) |

Der Feststoffanteil in der versprühten Lösungen betrug 10% (w/v) in 50 ml, 3,33% in 300 ml sowie 3,33% in 600 ml. Die Einlasstemperatur betrug ca. 170 bis 185°C, die Liquid-Feed-Rate ca. 3 ml/min, die Aspirator-Flow-Rate ca. 35 m³/h und die Zerstäubungsflussrate ca. 0,67 m³/h. Daraus ergibt sich eine Auslasstemperatur von ca. 80-95°C.

### Röntgendiffraktometrie (Weitwinkelröntaendiffraktometrie (WAXS)):

Um die Kristallinität der getrockneten Proben zu bestimmen, wurden die Proben mit einem Seifert, X- Ray Diffractometer XRD 3000 TT (Fa. Seifert, Ahrensburg, DE) in einem auf 22 °C temperierten Raum untersucht. Die Röntgenröhre Cu- Anode, Cu-Kα-Strahlung mit λ = 0,15418 mm (Primärfilter Ni), wurde mit einer Anodenspannung von 40 kV und einer Stromstärke von 30 mA betrieben. Nach Positionieren des Probentellers im Gerät wurde die Probe im Bereich von 5 bis 40° mit einer Scanrate von 2 θ = 0,05° mit 2 sec Messdauer bei jedem Winkel vermessen.

Die Pulverdiffraktogramme wurden mit der ScanX- Rayflex Applikation, Version 3.07 device XRD 3000 (Scan), bzw. der Rayflex Version 2.1, 1996 (Analyse) am Detektor SC 1000 V aufgenommen.

### Größenausschlusschromatographie (SEC-HPLC)

### a) Lösliche IgG1-Proteinaggregate

Um IgG1-Proteinaggregate in den rekonstituierten Pulvern zu quantifizieren wurde eine SEC-HPLC durchgeführt. Die SEC-HPLC wurde mit einer HP1090 der Firma Agilent durchgeführt. Zur Trennung wurde eine TSK3000SWXL Säule (300 x 7.8mm) der Fa. Tosoh Biosep (Tosoh Bioscience, Stuttgart, Deutschland) verwendet. Als Fließmittel wurde ein Puffer aus 0.1M di-Natriumhydrogenphosphat-Dihydrat, 0.1M Natriumsulfat entwässert und wurde mit ortho-Phosphorsäure 85% auf pH 6.8 eingestellt. Die aufgegebene Probenmenge betrug 25*µ*l bei einer Proteinkonzentration von 2-10 mg/ml. Der Nachweis des Proteins erfolgte mit Hilfe eines Dioden-Array Detektors der Firma Agilent bei 280nm. Für die Auswertung der Chromatogramme wurde die Software HP-Chemstation der Firma Agilent verwendet.

### b) Lösliche Calcitonin-Proteinaggregate

Um Calcitonin-Proteinaggregate in den rekonstituierten Pulvern zu quantifizieren wurde eine SEC-HPLC durchgeführt. Die SEC-HPLC wurde mit einer HP1100 der Firma Agilent durchgeführt. Zur Trennung wurde eine TSK2000SWXL Säule (300 x 7.8mm) der Fa. Tosoh Biosep (Tosoh Bioscience, Stuttgart, Deutschland) verwendet. Als Fließmittel wurde ein Puffer aus 0.25 Natriumsulfat mit einem pH von ca. 6 verwendet (Windisch et al. 1997). Die aufgegebene. Probenmenge betrug 20*µ*l bei einer Proteinkonzentration von 0,5-2 mg/ml. Der Nachweis des Proteins erfolgte mit Hilfe eines UV-Detektors der Firma Agilent bei 210nm. Für die Auswertung der Chromatogramme wurde die Software HP-Chemstation der Firma Agilent verwendet.

### c) Lysozym-Restmonomergehalt

Um den Lysozym-Restmonomergehalt in den rekonstituierten Lsozym-Formulierungen zu quantifizieren wurde eine modifizeirte SEC-HPLC (van de Weert, 2000) durchgeführt. Die SEC-HPLC wurde mit einer HP1100 der Firma Agilent durchgeführt.

Zur Trennung wurde eine TSK2000SWXL Säule (300 x 7.8mm) der Fa. Tosoh Biosep (Tosoh Bioscience, Stuttgart, Deutschland) verwendet. Als Fließmittel wurde ein Puffer aus 0,05 M di-Natriumhydrogenphosphat-Dihydrat und 0,2 M Natriumchlorid mit ortho-Phosphorsäure 85% auf pH 7.0 eingestellt. Die aufgegebene Probenmenge betrug 25*µ*l bei einer Proteinkonzentration von 2-10 mg/ml. Der Nachweis des Proteins erfolgte mit Hilfe eines UV-Detektors der Firma Agilent bei 280nm. Für die Auswertung der Chromatogramme wurde die Software Agilent Chemstation der Firma Agilent verwendet.

Zur Beurteilung der Formuierungen wird das verbliebene lösliche Monomer nach folgender Methode qauntifiziert. Zunächst wurde eine Eichgerade mit Lysozymstandardlösungen mit Konzentrationen von 2.5 mg/ml, 5.0 mg/ml und 10 mg/ml erstellt. Dabei wurde die AUC der Monomerpeaks in Relation zur den entsprechenden Lysozymkonzentrationen in den untersuchten Standardlösung betrachtet.

Der Restmonomergehalt der untersuchten verschiedenen Lysozymformulierungen wurde anhand der Eichgeraden errechnet. Je höher der Restmonomergehalt einer Formulierung ist, desto besser ist die Proteinstabilität.

### Partikelgrößenbestimmung (MMD)

Der Mass Median Diameter oder die mittlere Teilchengrößen der Partikel wurde mit Hilfe des Sympatech Helos der Fa Sympatech GmbH (Clausthal-Zellerfeld, DE) bestimmt. Das Messprinzip beruht auf Laserbeugung, verwendet wird ein Helium-Neon-Laser. 1-3 mg Pulver werden mit einem Luftdruck von 2bar dispergiert, und vor der Fourierlinse (50mm) durch einen parallelen Laserstrahl geführt. Die Partikelgrößenverteilung wird mit einem Fraunhofer-Modell ausgewertet. Pro Pulver wurden zwei Messungen durchgeführt.

### Mass Median Diameter (MMAD) und Feinpartikelfraktion (FPF)

Für die Messungen wurden jeweils 12-18mg Pulver in Hartgelatine Kapseln (Größe 3) abgefüllt und in den HandiHaler (Pulver-Inhalationsgerät der Firma Boehringer Ingelheim) eingebracht. Mit einem Adapter wurde der HandiHaler an den USP EP/Throat des Impaktorinlets des Messgerätes angekoppelt und das Pulver mit 39,0l/min bei einer Sogzeit von 6,15sec ausgebracht. Die Steuerung des Luftdurchsatzes erfolgte über eine externe Steuerwand. Für jedes Pulver wurden zumindest drei Kapseln vermessen.

Mit dem APS 3321 der Firma TSI Inc., MN, USA wird in Kombination mit dem Impaktorinlet 3306 simultan die aerodynamische Teilchengröße (MMAD) über eine Flugzeitbestimmung und die Feinpartikelfraktion (FPF) über einen Einstufenimpaktor (effektiver Cut off Diameter bei 39L/min: 5,0*µ*m) bestimmt. Das Pulver gelangt nach Ausbringung über den EP/USP Throat oder Sample Induction Port zu einer dünnen Kapillare, wo 0,2% der Pulvermenge für die Flugzeitmessung (Time of Flight) unter isokinetischen Bedingungen entnommen wird. Die Flugzeitmessung erfolgt nach dem Passieren der Kapillare über 2 Laserstrahlen, die analog einer Lichtschranke die Flugzeiten für eine definierte Strecke erfassen. Als Ergebnis erhält man eine Anzahlverteilung, die anschließend auf eine Massenverteilung und damit auf den Mass Median Aerodynamic Diameter (MMAD) umgerechnet wird.

Die restlichen 99,8% der Pulverpopulation, die an der Kapillare vorbeigeführt wurden, werden über den Einstufenimpaktor aufgetrennt. Die Fraktion größer 5,0 *µ*m scheidet sich im Impaktor aufgrund der Massenträgheit auf einer Prallplatte ab. Die Feinpartikelfraktion (FPF) folgt dem Luftstrom und wird schließlich auf einem Tiefenfilter abgeschieden. Die Bestimmung der Feimpartikelfraktion erfolgte gravimetrisch. Die Feinpartikelfraktion berechnet sich aus dem Anteil des auf dem Filter abgeschiedenen Pulvers in Bezug auf die Gesamtmenge an eingesetztem Pulver, d.h. eingewogenes Pulver pro Kapsel.

### Restwassergehalt:

Der Restwassergehalt in den getrockneten Produkten wurde mittels coulometrischer Titration (Metrohm 737 KF Coulometer mit 703 Titrationsstand, Deutschland) bestimmt. Zur Bestimmung wurde Pulver in Methanol (Hydranal - Methanol dry, VWR / Merck Eurolab) aufgelöst bzw. dispergiert. Die Messlösung (Hydranal -Coulomat-Lösung, VWR / Merck Eurolab) des Metrohm Coulometers wurde zu Beginn der Messungen konditioniert, d.h. die Messlösung wurde auf einen Nullgehalt an Wasser austariert. Die Probe wurde in die Titrationszelle injiziert und vermessen.

### Stabilitätsbestimmung:

Die Pulver wurden nach der Sprühtrocknung auf verschiedene Stabilitäten untersucht. Bei IgG1 und Calcitonin wurde als Maß für die Stabilität der Formulierungen der prozentuale Anteil der Proteinaggregate gewertet. Bei Lysozym wurde als Maß für die Stabilität der Formulierungen der prozentuale Anteil des Restmonomergehltes gewertet. Den in der Erfindung beschriebenen innovativen Hilfsstoffen wurde als Referenz die reine Proteinformulierung und gegebenenfalls eine analoge Trehalose-Formulierung gegenüber gestellt. Die Analytik zur Untersuchung auf Aggregate wurde mit einer validierten Größenausschlusschromatographie (SEC-HPLC) mit UV-Detektion (DAD) durchgeführt. Dazu wurden die vorbehandelten Pulver zunächst in Reinstwasser (pH von 6 bis 8) rekonstituiert.

Ausgewählte Formulierungen wurden nach einwöchiger offener Lagerung bei ca. 40°C und ca. 75% relativer Luftfeuchtigkeit (40°C, 75% rF) in offenen Glasvials auf ihre Stabilität untersucht (Forcierte Lagerstabilität).

Ausgewählte Formulierungen wurden nach Sprühtrocknung und Vakuumtrocknung unter Stickstoff in geschlossen Glasvials bei 2-8°C, 25°C sowie 40°C gelagert. Die Formulierungen wurden nach einem, drei, sechs und zwölf Monaten entnommen und auf Ihre Stabilität untersucht (1 Jahresstabilität).

### Beispiel 1 (nicht erfindungsgemäß)

### Sprühtrocknung einer IgG1 10% (w/v) Formulierung

Pures IgG1 mit eine Konzentration von ca. 109 mg/ml, formuliert in einem Glycin Histidin Puffer pH 6 (siehe Materialien), wurde mit demineralisiertem Wasser (pH ca. 7,5) auf einen Gehalt von 100mg/ml verdünnt und in Abwesenheit von weiteren Hilfsstoffen wie oben beschrieben unter Verwendung des Zyklon I sprühgetrocknet. Das Volumen der Lösung umfasste 50 ml. Der Gehalt an Aggregaten wurde wie oben beschrieben untersucht. Nach forcierter Lagerung hatte die Lösung aus dem rekonstituierten Pulver ca. 18,9% Aggregate

### Sprühtrocknung einer Trehalose 9% (w/v) IgG1 1% (w/v) Formulierung

4,5 g Trehalose wurde in ca. 40 ml demineralisiertem Wasser (pH ca. 7,5) gelöst. Als nächstes wurden ca. 4,6 ml pures IgG1 mit eine Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I sprühgetrocknet. Der Gehalt an Aggregaten wurde wie oben beschrieben untersucht. Nach forcierter Lagerung hatte die Lösung aus dem rekonstituierten Pulver ca. 12,6% Aggregate.

### Sprühtrocknung einer Dextran₁₀₀₀ 9% (w/v) IgG1 1% (w/v) Formulierung

4,5 g Dextran₁₀₀₀ wurde in ca. 40 ml demineralisiertem Wasser (pH ca. 7,5) gelöst. Als nächstes wurden ca. 4,6 ml pures IgG1 mit eine Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I sprühgetrocknet. Der Gehalt an Aggregaten wurde wie oben beschrieben untersucht. Für die Lagerstabilität ergaben sich folgende Aggregatgehalte. Nach forcierter Lagerung hatte die Lösung aus dem rekonstituierten Pulver ca. 5,1% Aggregate.

### Sprühtrocknung einer Dextran₁₀₀₀ 2,00% (w/v) IgG1 1,33% (w/v) Formulierung

3,0 g Dextran₁₀₀₀ wurde in ca. 120 ml demineralisiertem Wasser (pH ca. 7,5) gelöst. Als nächstes wurden ca. 19,5 ml pures IgG1 mit eine Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 150 ml verdünnt. Die so erhaltene Lösung enthält ca. 2,0% (w/v) Hilfsstoff oder Matrix sowie 1,33% (w/v) Protein und wurde wie oben beschrieben unter Verwendung des Zyklon II sprühgetrocknet. Der Gehalt an Aggregaten wurde wie oben beschrieben untersucht. Für die Lagerstabilität ergaben sich folgende Aggregatgehalte. Nach forcierter Lagerung hatte die Lösung aus dem rekonstituierten Pulver ca. 11,1% Aggregate.

### Beispiel 2 (nicht erfindungsgemäß)

### Sprühtrocknung einer Trehalose 8% (w/v) L-Isoleucin 1% (w/v) IgG1 1% (w/v) Formulierung

4 g Trehalose und 0,5 g L-Isoleucin wurden im Ultraschallbad in ca. 40 ml demineralisiertem Wasser (pH ca. 7,5) gelöst. Als nächstes wurden ca. 4,6 ml pures IgG1 mit eine Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I sprühgetrocknet. Der Gehalt an Aggregaten wurde wie oben beschrieben untersucht. Nach forcierter Lagerung hatte die Lösung aus dem rekonstituierten Pulver ca. 22,2% Aggregate.

### Sprühtrocknung einer Dextran₁₀₀₀ 8% (w/v) L-Isoleucin 1% (w/v) IgG1 1% (w/v) Formulierung

4 g Dextran₁₀₀₀ und 0,5 g L-Isoleucin wurden im Ultraschallbad in ca. 40 ml demineralisiertem Wasser (pH ca. 7,5) gelöst. Als nächstes wurden ca. 4,6 ml pures IgG1 mit eine Konzentration von ca. 109 mg/ml, formuliert in einem Glycin Histidin Puffer pH 6 (siehe Materialien), zugegeben und mit demineralisiertem Wasser (pH ca. 7,5) auf ein Volumen von 50 ml verdünnt. Die so erhaltene Lösung enthält ca. 9% (w/v) Hilfsstoff oder Matrix sowie 1% (w/v) Protein und wurde wie oben beschrieben unter Verwendung des Zyklon I sprühgetrocknet. Der Gehalt an Aggregaten wurde wie oben beschrieben untersucht. Nach forcierter Lagerung hatte die Lösung aus dem rekonstituierten Pulver lediglich ca. 10,1% Aggregate. In einem zweiten größeren Ansatz wurde eine analoge Formulierung (20g Feststoff und 200 ml Volumen) bei gleichen Bedinungen sprühgetrocknet. Der Gehalt an Aggregaten wurde wie oben beschrieben untersucht. Nach 12 Monaten Lagerung bei 40°C (1 Jahresstabilität) hatte die Lösung aus dem rekonstituierten Pulver ca. 2,5% Aggregate. Nach 3 Monaten Lagerung bei 25°C (3 Monatsstabilität) hatte die Lösung aus dem rekonstituierten Pulver ca. 2,2% Aggregate. Nach 3 Monaten Lagerung bei 2-8°C (3 Monatsstabilität) hatten die Lösung aus dem rekonstituierten Pulver ca. 2,0% Aggregate. Das erhaltene Pulver wurde bezüglich MMD, MMAD und FPF vermessen. Der MMD des Pulvers wurde wie oben beschrieben bestimmt. Der MMD des Pulvers lag bei 5,11 *µ*m. Der MMAD und FPF des Pulvers wurde wie oben beschrieben bestimmt. Der MMAD lag bei 6,8 *µ*m und die Fine Particle Fraction lag bei 34,8 % bezogen auf die Pulvereinwaage in der Kapsel.

### Sprühtrocknung einer Dextran₁₀₀₀ 2,833% (w/v) L-Isoleucin 0,166% (w/v) IgG1 0,33% (w/v) Formulierung

8,5 g Dextran₁₀₀₀ und 0,5 g L-Isoleucin wurden im Ultraschallbad in ca. 280 ml demineralisiertem Wasser (pH ca. 7,5) gelöst. Als nächstes wurden ca. 9,7 ml pures IgG1 mit eine Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II sprühgetrocknet.

Der Gehalt an Aggregaten wurde wie oben beschrieben untersucht. Nach forcierter Lagerung hatte die Lösung aus dem rekonstituierten Pulver ca. 6,3% Aggregate.Der MMD des Pulvers wurde wie oben beschrieben bestimmt. Der MMD des Pulvers lag bei 2,98 *µ*m. Der MMAD und FPF des Pulvers wurde wie oben beschrieben bestimmt. Der MMAD lag bei 5,3 *µ*m und die Fine Particle Fraction lag bei 35,2 % bezogen auf die Pulvereinwaage in der Kapsel.

### Sprühtrocknung einer Dextran₁₀₀₀ 2,66% (w/v) L-Isoleucin 0,33% (w/v) IgG1 0,33% (w/v) Formulierung

8,0 g Dextran₁₀₀₀ und 1 g L-Isoleucin wurden im Ultraschallbad in ca. 280 ml demineralisiertem Wasser (pH ca. 7,5) gelöst. Als nächstes wurden ca. 9,7 ml pures IgG1 mit eine Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II sprühgetrocknet.

Der Gehalt an Aggregaten wurde wie oben beschrieben untersucht. Nach forcierter Lagerung hatte die Lösung aus dem rekonstituierten Pulver ca. 7,1% Aggregate. Nach 12 Monaten Lagerung bei 40°C (1 Jahresstabilität) hatte die Lösung aus dem rekonstituierten Pulver ca. 3,3% Aggregate. Nach 12 Monaten Lagerung bei 25°C (1 Jahresstabilität) hatte die Lösung aus dem rekonstituierten Pulver ca. 2,3% Aggregate. Nach 12 Monaten Lagerung bei 2-8°C (1 Jahresstabilität) hatten die Lösung aus dem rekonstituierten Pulver ca. 1,9% Aggregate. Der MMD des Pulvers wurde wie oben beschrieben bestimmt. Der MMD des Pulvers lag bei 2,75 *µ*m. Der MMAD und FPF des Pulvers wurde wie oben beschrieben bestimmt. Der MMAD lag bei 5,3 *µ*m und die Fine Particle Fraction lag bei 39,2 % bezogen auf die Pulvereinwaage in der Kapsel.

### Sprühtrocknung einer Dextran₁₀₀₀ 2,33% (w/v) L-Isoleucin 0,66% (w/v) IgG1 0,33% (w/v) Formulierung

7,0 g Dextran₁₀₀₀ und 2 g L-Isoleucin wurden im Ultraschallbad in ca. 280 ml demineralisiertem Wasser (pH ca. 7,5) gelöst. Als nächstes wurden ca. 9,7 ml pures IgG1 mit eine Konzentration von ca. 102,8 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II sprühgetrocknet.

Der Gehalt an Aggregaten wurde wie oben beschrieben untersucht. Nach forcierter Lagerung hatte die Lösung aus dem rekonstituierten Pulver ca. 10,6% Aggregate. Der MMD des Pulvers wurde wie oben beschrieben bestimmt. Der MMD des Pulvers lag bei 2,71 *µ*m. Der MMAD und FPF des Pulvers wurde wie oben beschrieben bestimmt. Der MMAD lag bei 5,1 *µ*m und die Fine Particle Fraction lag bei 36,4 % bezogen auf die Pulvereinwaage in der Kapsel.

### Beispiel 3

### Sprühtrocknung einer Dextran₁₀₀₀ 2,66% (w/v) Tri-Isoleucin 0,33% (w/v) IgG1 0,33% (w/v) Formulierung

16,0 g Dextran₁₀₀₀ und 2 g Tri-Isoleucin wurden im Ultraschallbad in ca. 560 ml demineralisiertem Wasser (pH ca. 7,5) gelöst. Als nächstes wurden ca. 20,7 ml pures IgG1 mit eine Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 600 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon I sprühgetrocknet.

Der Gehalt an Aggregaten wurde wie oben beschrieben untersucht. Nach 3 Monaten Lagerung bei 40°C (3 Monatsstabilität) hatte die Lösung aus dem rekonstituierten Pulver ca 3,2% Aggregate. Nach 3 Monaten Lagerung bei 25°C (3 Monatsstabilität) hatte die Lösung aus dem rekonstituierten Pulver ca 1,2% Aggregate. Nach 3 Monaten Lagerung bei 2-8°C (3 Monatsstabilität) hatten die Lösung aus dem rekonstituierten Pulver ca. 0,9% Aggregate. Nach 12 Monaten Lagerung bei 40°C (1 Jahresstabilität) hatte die Lösung aus dem rekonstituierten Pulver ca. 7,3% Aggregate. Nach 12 Monaten Lagerung bei 25°C (1 Jahresstabilität) hatte die Lösung aus dem rekonstituierten Pulver ca. 2,0% Aggregate. Nach 12 Monaten Lagerung bei 2-8°C (1 Jahresstabilität) hatten die Lösung aus dem rekonstituierten Pulver ca. 1,3% Aggregate. Der MMAD und FPF des Pulvers wurde wie oben beschrieben bestimmt. Der MMAD lag bei 4,6 *µ*m und die Fine Particle Fraction lag bei 55,7% bezogen auf die Pulvereinwaage in der Kapsel.

### Sprühtrocknung einer Dextran₁₀₀₀ 2,66% (w/v) Tri-Isoleucin 0,33% (w/v) IgG1 0,33% (w/v) Formulierung

8,0 g Dextran₁₀₀₀ und 1 g Tri-Isoleucin wurden im Ultraschallbad in ca. 280 ml demineralisiertem Wasser (pH ca. 7,5) gelöst. Als nächstes wurden ca. 10,36 ml pures IgG1 mit eine Konzentration von ca. 96,55 mg/ml, formuliert in einem Glycin Histidin Puffer pH 6 (siehe Materialien), zugegeben und mit demineralisiertem Wasser (pH ca. 7,5) auf ein Volumen von 300 ml verdünnt. Die so erhaltene Lösung enthält ca. 3% (w/v) Hilfsstoff oder Matrix sowie 0,33% (w/v) Protein und wurde wie oben beschrieben unter Verwendung des Zyklon II sprühgetrocknet.

Eine Untersuchung der Stabilität wurde nicht vorgenommen, da die Formulierung exakt mit der direkt oberen übereinstimmt. Die Verwendung eines anderen Zyklons hat keinen Einfluss auf die Proteinstabilität. Der MMD des Pulvers wurde wie oben beschrieben bestimmt. Der MMD des Pulvers lag bei 2,96 *µ*m. Der MMAD und FPF des Pulvers wurde wie oben beschrieben bestimmt. Der MMAD lag bei 3,9 *µ*m und die Fine Particle Fraction lag bei 58,4 % bezogen auf die Pulvereinwaage in der Kapsel.

### Beispiel 4: Herstellung von weiteren erfindungsgemäßen Pulvern

### Sprühtrocknung einer Lysozym 3,33% (w/v) Formulierung

5 g Lysozym wird in ca. 140 ml demineralisiertem Wasser (pH ca. 7,5) gelöst und mit demineralisiertem Wasser (pH ca. 7,5) auf ein Volumen von 150ml verdünnt. Die so erhaltene Lösung wird wie oben beschrieben unter Verwendung des Zyklon II sprühgetrocknet.

Der Restmonomergehalt wurde wie oben beschrieben untersucht. Nach forcierter Lagerung hatte die Lösung aus dem rekonstituierten Pulver einen Restmonomergehalt von 35,3%. Der MMD des Pulvers wurde wie oben beschrieben bestimmt. Der MMD des Pulvers lag bei 3,23 *µ*m. Der MMAD und FPF des Pulvers wurde wie oben beschrieben bestimmt. Der MMAD lag bei 4,0 *µ*m und die Fine Particle Fraction lag bei 70,4 % bezogen auf die Pulvereinwaage in der Kapsel.

### Sprühtrocknung einer Dextran₁₀₀₀ 3,00% (w/v) Lysozym 0,33% (w/v) Formulierung

9,0 g Dextran₁₀₀₀ wird im Ultraschallbad in ca. 280 ml demineralisiertem Wasser (pH ca. 7,5) gelöst. Als nächstes wird 1 g Lysozym, und mit demineralisiertem Wasser (pH ca. 7,5) auf ein Volumen von 300ml verdünnt. Die so erhaltene Lösung wird wie oben beschrieben unter Verwendung des Zyklon II sprühgetrocknet.

Der Restmonomergehalt wurde wie oben beschrieben untersucht. Nach forcierter Lagerung hatte die Lösung aus dem rekonstituierten Pulver einen Restmonomergehalt von 49,8%. Der MMD des Pulvers wurde wie oben beschrieben bestimmt. Der MMD des Pulvers lag bei 2,82 *µ*m. Der MMAD und FPF des Pulvers wurde wie oben beschrieben bestimmt. Der MMAD lag bei 4,2 *µ*m und die Fine Particle Fraction lag bei 34,7 % bezogen auf die Pulvereinwaage in der Kapsel.

### Sprühtrocknung einer Dextran₁₀₀₀ 2,66% (w/v) Isoleucin 0,33 % (w/v) Lysozym 0,33% (w/v) Formulierung

8,0 g Dextran₁₀₀ und 1 g Isoleucin werden im Ultraschallbad in ca. 280 ml demineralisiertem Wasser (pH ca. 7,5) gelöst. Als nächstes wird 1 g Lysozym, und mit demineralisiertem Wasser (pH ca. 7,5) auf ein Volumen von 300ml verdünnt. Die so erhaltene Lösung wird wie oben beschrieben unter Verwendung des Zyklon II sprühgetrocknet.

Der Restmonomergehalt wurde wie oben beschrieben untersucht. Nach forcierter Lagerung hatte die Lösung aus dem rekonstituierten Pulver einen Restmonomergehalt von 50,7%. Der MMD des Pulvers wurde wie oben beschrieben bestimmt. Der MMD des Pulvers lag bei 3,01 *µ*m. Der MMAD und FPF des Pulvers wurde wie oben beschrieben bestimmt. Der MMAD lag bei 4,2 *µ*m und die Fine Particle Fraction lag bei 36,6 % bezogen auf die Pulvereinwaage in der Kapsel.

### Sprühtrocknung einer Dextran₁₀₀₀ 2,66% (w/v) Tri-Isoleucin 0,33 % (w/v) Lysozym 0,33% (w/v) Formulierung

8,0 g Dextran₁₀₀₀ und 1 g Tri-Isoleucin werden im Ultraschallbad in ca. 280 ml demineralisiertem Wasser (pH ca. 7,5) gelöst. Als nächstes wird 1 g Lysozym, zugegeben und mit demineralisiertem Wasser (pH ca. 7,5) auf ein Volumen von 300ml verdünnt. Die so erhaltene Lösung wird wie oben beschrieben unter Verwendung des Zyklon II sprühgetrocknet.

Der Restmonomergehalt wurde wie oben beschrieben untersucht. Nach forcierter Lagerung hatte die Lösung aus dem rekonstituierten Pulver einen Restmonomergehalt von 43,9%. Der MMD des Pulvers wurde wie oben beschrieben bestimmt. Der MMD des Pulvers lag bei 2,53 *µ*m. Der MMAD und FPF des Pulvers wurde wie oben beschrieben bestimmt. Der MMAD lag bei 3,2 *µ*m und die Fine Particle Fraction lag bei 58,6 % bezogen auf die Pulvereinwaage in der Kapsel.

### Sprühtrocknung einer Calcitonin 3,33% (w/v) Formulierung

1 g Calcitonin wird in ca. 25 ml demineralisiertem Wasser (pH ca. 7,5) gelöst und mit demineralisiertem Wasser (pH ca. 7,5) auf ein Volumen von 30ml verdünnt. Die so erhaltene Lösung wird wie oben beschrieben unter Verwendung des Zyklon II sprühgetrocknet.

Der Gehalt an Aggregaten wurde wie oben beschrieben untersucht. Nach forcierter Lagerung hatte die Lösung aus dem rekonstituierten Pulver ca. 32,6 % Aggregate. Der MMAD und FPF des Pulvers wurde wie oben beschrieben bestimmt. Der MMAD lag bei 3,9 *µ*m und die Fine Particle Fraction lag bei 59,0 % bezogen auf die Pulvereinwaage in der Kapsel.

### Sprühtrocknung einer Dextran₁₀₀₀ 3,166% (w/v) Calcitonin 0,166% (w/v) Formulierung

4,750 g Dextran₁₀₀₀ wird im Ultraschallbad in ca. 140 ml demineralisiertem Wasser (pH ca. 7,5) gelöst. Als nächstes wird 0,250 g Calcitonin zugegeben und mit demineralisiertem Wasser (pH ca. 7,5) auf ein Volumen von 150 ml verdünnt. Die so erhaltene Lösung wird wie oben beschrieben unter Verwendung des Zyklon II sprühgetrocknet.

Der Gehalt an Aggregaten wurde wie oben beschrieben untersucht. Nach forcierter Lagerung hatte die Lösung aus dem rekonstituierten Pulver ca. 27,5 % Aggregate. Der MMD des Pulvers wurde wie oben beschrieben bestimmt. Der MMD des Pulvers lag bei 3,00 *µ*m. Der MMAD und FPF des Pulvers wurde wie oben beschrieben bestimmt. Der MMAD lag bei 4,6 *µ*m und die Fine Particle Fraction lag bei 42,6 % bezogen auf die Pulvereinwaage in der Kapsel.

### Sprühtrocknung einer Dextran₁₀₀₀ 2,833% (w/v) Isoleucin 0,33 % (w/v) Calcitonin 0,166% (w/v) Formulierung

4,250 g Dextran₁₀₀₀ und 0,50 g Isoleucin werden im Ultraschallbad in ca. 140 ml demineralisiertem Wasser (pH ca. 7,5) gelöst. Als nächstes wird 0,250 g Calcitonin zugegeben und mit demineralisiertem Wasser (pH ca. 7,5) auf ein Volumen von 150 ml verdünnt. Die so erhaltene Lösung wird wie oben beschrieben unter Verwendung des Zyklon II sprühgetrocknet.

Der Gehalt an Aggregaten wurde wie oben beschrieben untersucht. Nach forcierter Lagerung hatte die Lösung aus dem rekonstituierten Pulver ca. 23,2 % Aggregate. Der MMD des Pulvers wurde wie oben beschrieben bestimmt. Der MMD des Pulvers lag bei 2,86 *µ*m. Der MMAD und FPF des Pulvers wurde wie oben beschrieben bestimmt. Der MMAD lag bei 4,5 *µ*m und die Fine Particle Fraction lag bei 57,1 % bezogen auf die Pulvereinwaage in der Kapsel.

### Sprühtrocknung einer Dextran₁₀₀₀ 2,866% (w/v) Tri-Isoleucin 0,33 % (w/v) Calcitonin 0,166% (w/v) Formulierung

4,250 g Dextran₁₀₀₀ und 0,50 g Tri-Isoleucin werden im Ultraschallbad in ca. 140 ml demineralisiertem Wasser (pH ca. 7,5) gelöst. Als nächstes wird 0,250 g Calcitonin zugegeben und mit demineralisiertem Wasser (pH ca. 7,5) auf ein Volumen von 150 ml verdünnt. Die so erhaltene Lösung wird wie oben beschrieben unter Verwendung des Zyklon II sprühgetrocknet.

Der MMD des Pulvers wurde wie oben beschrieben bestimmt. Der MMD des Pulvers lag bei 2,60 *µ*m. Der MMAD und FPF des Pulvers wurde wie oben beschrieben bestimmt. Der MMAD lag bei 3,7 *µ*m und die Fine Particle Fraction lag bei 62,5% bezogen auf die Pulvereinwaage in der Kapsel.

## Patentansprüche

1. Sprühgetrocknetes Pulver enthaltend einen pharmazeutischen Wirkstoff, ein Tri-Isoleucin und ein niedermolekulares Dextran mit einem Molekulargewicht zwischen 500 und 10.000 Da.

2. Sprühgetrocknetes Pulver gemäß Anspruch 1 ***dadurch gekennzeichnet,* dass** es sich bei dem pharmazeutischen Wirkstoff um ein biologisches Makromolekül handelt.

3. Sprühgetrocknetes Pulver nach einem der Ansprüche 1 bis 2 ***dadurch gekennzeichnet,* dass** der Anteil an pharmazeutischem Wirkstoff zwischen 0,01 und 50% (w/w) der Trockenmasse des Pulvers beträgt.

4. Sprühgetrocknetes Pulver nach einem der Ansprüche 1 bis 3 **dadurch gekennzeichnet, dass** das sprühgetrocknete Pulver ein oder mehrere pharmazeutisch verträgliche Hilfsstoffe und/oder ein oder mehrere Salze enthält.

5. Sprühgetrocknetes Pulver nach einem der Ansprüche 1 bis 4 ***dadurch gekennzeichnet,* dass** die Partikel im Pulver über einen "Mass Mean Aerodynamic Diameter" zwischen 1 und 10 µm verfügen.

6. Sprühgetrocknetes Pulver nach einem der Ansprüche 1 bis 5 ***dadurch gekennzeichnet,* dass** das sprühgetrocknete Pulver amorph ist.

7. Pharmazeutische Zusammensetzung enthaltend ein sprühgetrocknetes Pulver nach einem der Ansprüche 1 bis 6.

8. Verfahren zur Herstellung eines sprühgetrockneten Pulvers gemäß Anspruch 1 bis 6 ***dadurch gekennzeichnet,* dass**
a) ein pharmazeutischer Wirkstoff in einer wässrigen Lösung / Suspension gelöst wird;
b) niedermolekulares Dextran in einer wässrigen Lösung / Suspension gelöst wird;
c) die Lösung oder Suspension neben dem niedermolekularen Dextran und dem pharmazeutischen Wirkstoff zumindest ein Tri-Isoleucin als Hilfsstoff enthält und sofern Wirkstoff, Tri-Isoleucin und niedermolekulares Dextran in verschiedenen Lösungen / Suspension gelöst wird, diese gemischt werden;
d) die Lösung / Suspension enthaltend niedermolekulares Dextran und den pharmazeutischen Wirkstoff unterhalb von einer Temperatur von 200/120°C (Einström- /Ausströmtemperatur), vorzugsweise von 150-185/80-95°C versprüht wird.

9. Verfahren nach Anspruch 8 **dadurch gekennzeichnet, dass** der pharmazeutische Wirkstoff ein biologisches Makromolekül ist.

10. Verfahren nach einem der Ansprüche 8 bis 9 ***dadurch gekennzeichnet,* dass** die Lösung oder Suspension zusätzlich ein oder mehrere Hilfsstoffe und/oder ein oder mehrere Salze enthält.

11. Verfahren nach Anspruch 10 ***dadurch gekennzeichnet,* dass** die Lösung oder Suspension einen pH-Wert zwischen 3.0 und 9.0 aufweist.

12. Verfahren nach einem der Ansprüche 8 bis 11 ***dadurch gekennzeichnet,* dass** die Trockenmasse der Lösung oder der Suspension zumindest 50% (w/w) eines niedermolekularen Dextrans und zwischen 0,01 und 50% (w/w) eines biologischen Makromoleküls enthält.

13. Verfahren nach Anspruch 12 ***dadurch gekennzeichnet,* dass** Trockenmasse der Lösung oder der Suspension zumindest 50% (w/w) niedermolekulares Dextran und zwischen 1 und 20% (w/w) Tri-Isoleucin enthält.

14. Verwendung des sprühgetrockneten Pulvers nach einem der Ansprüche 1 bis 6 zur Herstellung eines Arzneimittels.

15. Verwendung des sprühgetrockneten Pulvers nach Anspruch 14 zur Herstellung eines inhalativen Arzneimittels.

## Claims

1. Spray-dried powder containing a pharmaceutical active ingredient, a tri-isoleucine and a low-molecular dextran having a molecular weight between 500 and 10,000 Da.

2. Spray-dried powder according to claim 1, **characterised in that** the pharmaceutical active ingredient is a biological macromolecule.

3. Spray-dried powder according to either claim 1 or claim 2, **characterised in that** the proportion of pharmaceutical active ingredient is between 0.01 and 50% (w/w) of the dry matter of the powder.

4. Spray-dried powder according to any one of claims 1 to 3, **characterised in that** the spray-dried powder contains one or more pharmaceutically compatible auxiliary substances and/or one or more salts.

5. Spray-dried powder according to any one of claims 1 to 4, **characterised in that** the particles in the powder have a mass mean aerodynamic diameter between 1 and 10 µm.

6. Spray-dried powder according to any one of claims 1 to 5, **characterised in that** the spray-dried powder is amorphous.

7. Pharmaceutical composition containing a spray-dried powder according to any of claims 1 to 6.

8. Method for producing a spray-dried powder according to claims 1 to 6, **characterised in that**
a) a pharmaceutical active ingredient is dissolved in an aqueous solution/suspension;
b) low-molecular dextran is dissolved in an aqueous solution/suspension;
c) the solution or suspension contains, in addition to the low-molecular dextran and the pharmaceutical active ingredient, at least one tri-isoleucine as an auxiliary substance, and if the active ingredient, tri-isoleucine and low-molecular dextran are dissolved in different solutions/suspensions, these are mixed;
d) the solution/suspension containing low-molecular dextran and the pharmaceutical active ingredient is sprayed below a temperature of 200/120°C (inflow/outflow temperature), preferably 150-185/80-95°C.

9. Method according to claim 8, **characterised in that** the pharmaceutical active ingredient is a biological macromolecule.

10. Method according to either claim 8 or claim 9, **characterised in that** the solution or suspension additionally contains one or more auxiliary substances and/or one or more salts.

11. Method according to claim 10, **characterised in that** the solution or suspension has a pH between 3.0 and 9.0.

12. Method according to any one of claims 8 to 11, **characterised in that** the dry matter of the solution or the suspension contains at least 50% (w/w) of a low-molecular dextran and between 0.01 and 50% (w/w) of a biological macromolecule.

13. Method according to claim 12, **characterised in that** the dry matter of the solution or the suspension contains at least 50% (w/w) low-molecular dextran and between 1 and 20% (w/w) tri-isoleucine.

14. Use of the spray-dried powder according to any one of claims 1 to 6 for producing a drug.

15. Use of the spray-dried powder according to claim 14 for producing an inhalable drug.

## Revendications

1. Poudre séchée par pulvérisation contenant un principe actif pharmaceutique, une tri-isoleucine et un dextrane à faible poids moléculaire avec un poids moléculaire compris entre 500 et 10.000 Da.

2. Poudre séchée par pulvérisation selon la revendication 1, **caractérisée en ce que** le principe actif pharmaceutique est une macromolécule biologique.

3. Poudre séchée par pulvérisation selon l'une quelconque des revendications 1 à 2, **caractérisée en ce que** la fraction en principe actif pharmaceutique présente une valeur comprise entre 0,01 et 50 % en poids de la matière sèche de la poudre.

4. Poudre séchée par pulvérisation selon l'une quelconque des revendications 1 à 3, **caractérisée en ce que** la poudre séchée par pulvérisation contient un ou plusieurs adjuvants pharmaceutiquement acceptables et/ou un ou plusieurs sels.

5. Poudre séchée par pulvérisation selon l'une quelconque des revendications 1 à 4, **caractérisée en ce que** les particules dans la poudre disposent d'un diamètre aérodynamique moyen en masse compris entre 1 et 10 µm.

6. Poudre séchée par pulvérisation selon l'une quelconque des revendications 1 à 5, **caractérisée en ce que** la poudre séchée par pulvérisation est amorphe.

7. Composition pharmaceutique contenant une poudre séchée par pulvérisation selon l'une quelconque des revendications 1 à 6.

8. Procédé servant à fabriquer une poudre séchée par pulvérisation selon la revendication 1 à 6,
**caractérisé en ce que**
a) un principe actif pharmaceutique est dissous dans une solution/suspension aqueuse ;
b) un dextrane à faible poids moléculaire est dissous dans une solution/suspension aqueuse ;
c) la solution ou suspension contient, outre le dextrane à faible poids moléculaire et le principe actif pharmaceutique, au moins une tri-isoleucine en tant qu'adjuvant et dans la mesure où le principe actif, la tri-isoleucine et le dextrane à faible poids moléculaire sont dissous dans différentes solutions/suspensions, ces derniers sont mélangés ;
d) la solution/suspension contenant un dextrane à faible poids moléculaire et le principe actif pharmaceutique est aspergée à une température inférieure à une température de 200/120 °C (température de flux entrant/de flux sortant), de préférence de 150-185/80-95 °C.

9. Procédé selon la revendication 8, **caractérisé en ce que** le principe actif pharmaceutique est une macromolécule biologique.

10. Procédé selon l'une quelconque des revendications 8 à 9, **caractérisé en ce que** la solution ou suspension contient en supplément un ou plusieurs adjuvants et/ou un ou plusieurs sels.

11. Procédé selon la revendication 10, **caractérisé en ce que** la solution ou suspension présente une valeur pH comprise entre 3,0 et 9,0.

12. Procédé selon l'une quelconque des revendications 8 à 11, **caractérisé en ce que** la matière sèche de la solution ou de la suspension contient au moins 50 % en poids d'un dextrane à faible poids moléculaire et entre 0,01 et 50 % en poids d'une macromolécule biologique.

13. Procédé selon la revendication 12, **caractérisé en ce que** la matière sèche de la solution ou de la suspension contient au moins 50 % en poids d'un dextrane à faible poids moléculaire et entre 1 et 20 % en poids d'une tri-isoleucine.

14. Utilisation de la poudre séchée par pulvérisation selon l'une quelconque des revendications 1 à 6 pour fabriquer un médicament.

15. Utilisation de la poudre séchée par pulvérisation selon la revendication 14 pour fabriquer un médicament à inhaler.
